# EUROPEAN PATENT APPLICATION

(11) **EP 4 534 076 A1**
(43) Date of publication of application: **09.04.2025**
(21) Application number: 24203526.9
(22) Date of filing: 30.09.2024
(51) Int. Cl.: A61K 9/20, A61K 9/28, A61K 31/167, A61K 31/415, A61K 45/06

(54) **CELECOXIB - ACETAMINOPHEN COMBINATION OF IMPROVED STABILITY AND PROCEDURE FOR THE PREPARATION THEREOF**

(30) Priority: 05.10.2023 MX 2023011800
(71) Applicant: Laboratorios Silanes, S.A. de C.V., Mexico, 11000 (MX)
(72) Inventor: González Canudas, Jorge Alejandro, 11000 Ciudad de México (MX); Espinosa Olivares, Marco Antonio, 11000 Ciudad de México (MX); Muñoz Martínez, Cecilia Jannette, 11000 Ciudad de México (MX); Ollervides Rubio, Paola Yazmín, 11000 Ciudad de México (MX); Sander Padilla, Guillermo, 11000 Ciudad de México (MX)
(74) Representative: Hoffmann Eitle

(57) **Abstract**

It is described and claimed a pharmaceutical composition comprising celecoxib and acetaminophen as active ingredients, wherein the pharmaceutical composition is an immediate release coated tablet. The pharmaceutical composition of the invention is particularly stable and allows the oral administration in a single dosage unit of two active ingredients. It is further provided a process for manufacturing the pharmaceutical composition, as well as the use of said pharmaceutical composition for the treatment of pain, it provides analgesia and a reduction of adverse effects.

## Description

### FIELD OF THE INVENTION

The present invention belongs to the field of pharmaceutical chemistry, particularly it relates to the combination of two active ingredients, celecoxib and acetaminophen in a solid pharmaceutical composition with immediate release and improved stability, for the treatment of pain; as well as to the manufacturing method that allows obtaining it, ensuring the chemical characteristics of each drug in the medicament and guaranteeing the stability of the product.

### BACKGROUND OF THE INVENTION

The compound 4-[5-(4-methylphenyl)-3-trifluoromethyl)-1H-pyrazol-1-yl]-benzenesulfonamide hereinafter referred to as celecoxib, was previously reported in US Patent 5,466,823 to TALLEY JOHN J, published on 11/14/1995, in which a group of 1,5-diarylpyrazoles and their salts is described and claimed. The reported 1,5-diarylpyrazole compounds are described as useful in the treatment of inflammation-related disorders. Said American patent has an equivalent in Mexico; this is the Mexican patent MX 200516, whose owner is G. D. SEARLE LLC; in this patent document protection was provided to the active ingredient celecoxib and its pharmaceutically acceptable salts.

According to Mexican patent MX 238788, published on 06/28/2001, many selective COX-2 inhibitor drugs, including celecoxib are hydrophobic and have low water solubility. This has presented practical difficulties in formulating oral administration compositions containing it, particularly when rapid onset of therapeutic effect is desired or required.

Formulation of celecoxib for effective oral administration to a subject has heretofore been complicated by the unique physical and chemical properties of celecoxib, particularly its low solubility and factors related to its crystal structure, including cohesiveness, low bulk density, and low compressibility. Celecoxib is unusually insoluble in aqueous media (therefore, it belongs to group II of the biopharmaceutical classification; due to its high permeability and low solubility). Furthermore, unformulated celecoxib has a crystal morphology that tends to form long cohesive needles; it typically coalesces into a monolithic mass upon compression to form tablets. Even when combined with other substances, celecoxib crystals tend to separate from the other substances, and agglomerate during mixing of the composition, resulting in a non-uniformly combined composition containing undesirable large aggregates of celecoxib. In response to the need for solutions to the various problems described in relation to celecoxib, said patent MX 238788 provides a formulation that includes one or more dual-release dosage units containing celecoxib as the sole API.

In addition, there are various marketable products in Mexico that contain celecoxib, for example, the reference medicament Celebrex^{®} which registered owner is Pfizer, S. A. de C. V., its marketing authorization number is 031M99 SSA. It is sold in pharmaceutical form of capsules in celecoxib concentrations of 100 mg, 200 mg, and 400 mg. LABORATORIOS SILANES S. A. DE C. V. is also the holder of the medicament Bienuex^{®}; its marketing authorization number is 162M2020 SSA under the pharmaceutical form of capsules in celecoxib concentrations of 100 mg or 200 mg.

On the other hand, the active ingredient acetaminophen, which according to the Merck index (Maryadele J. O'Neil; Patricia Heckelman. 15th edition. The Royal Society of Chemistry. 2013.) and the website: https://pubchem.ncbi.nlm.nih.gov/compound/1983, is also known as N-(4-hydroxyphenyl)acetamide; p-hydroxyacetanilide; p-acetamidophenol; N-acetyl-p-amino phenol; p-acetylaminophenol, APAP, or paracetamol. Additionally, it is relevant to mention that this active ingredient belongs to group III of the biopharmaceutical classification due to its high solubility and low permeability.

There are various marketable products in Mexico that contain acetaminophen; for example, the reference drug MEJORALITO PEDIATRICO^{®} which registered owner is the company GLAXOSMITHKLINE CONSUMER HEALTHCARE MEXICO, S. DE R. L. DE C. V., its marketing authorization number is 016M88 SSA and it is sold in 80 mg tablets. Regarding this active ingredient, the American patent US 2,998,450 of WARNER LAMBERT PHARMACEUTICAL can be cited, published on 08/29/1961, which provided protection for the first time to a process to produce N-acetyl-p-amino phenol. In the Chinese patent application CN201810166169 of CHONGQING HILAN PHARMACEUTICAL CO LTD, published on 13/07/2018, it is mentioned that acetaminophen tablets have poor stability and after long-term storage their intrinsic properties are reduced, for example, the disintegration effect decreases, and their dissolution becomes poor. Successively, the patent MX 332009 of TROIKAA PHARMACEUTICALS LIMITED, published on 01/05/2012, mentions that the compound p-acetylaminophenol, is an analgesic and antipyretic drug used to relieve fever, headaches, and other minor pains, it is a main ingredient in numerous medications for the flu, cold and many prescription pain relievers.

Based on the above, the combination of celecoxib with acetaminophen has been suggested because it has a particularly beneficial effect as an analgesic, since the sum of the analgesia obtained with the association of both active ingredients is greater than that obtained independently by each of them, this derived from the different mechanisms of action of each drug. On the one hand, celecoxib acts as a selective inhibitor of cyclooxygenase 2 (COX-2) and thus reduces the synthesis of prostaglandins, while acetaminophen acts primarily on the central nervous system by increasing the pain threshold by inhibiting an isoform of COX known as COX3, although the mechanism of this action is not yet fully described. The antipyretic properties of acetaminophen are due to direct effects on the temperature regulation center of the hypothalamus. Such is the case of patent MX366118 of More Pharma Corporation S. de RL de C. V., published on 01/15/2015, which protects a combination of acetaminophen and celecoxib that in specific amounts generates an analgesic potentiation against inflammatory pain. Specifically, said patent refers to a synergistic combination of 375 mg of acetaminophen with 50 to 75 mg of celecoxib, useful for the treatment of inflammatory pain in animals and humans, as well as the use of said active ingredients in the indicated amounts in the preparation of synergistic pharmaceutical combinations. As can be seen, patent MX366118 is aimed at obtaining a combination of acetaminophen and celecoxib that exhibits a synergistic effect, such as to reduce the doses of the active ingredients due to their known adverse effect on the gastrointestinal system. However, it does not show any interest in maintaining or ensuring the characteristics or chemical attributes of each drug in the medicament, to avoid chemical interactions that affect the stability of the product.

Continuing, there are other patent documents in the state of the art that refer to the combination of celecoxib with acetaminophen; however, these do not solve, or even contemplate, the technical problem involved in combining two drugs with different rheological and solubility characteristics, since on the one hand there is a liposoluble drug, with poor flow and excessive adhesiveness (celecoxib); and on the other, there is a hydrosoluble drug with poor flow and low compaction (acetaminophen); these properties could cause interference not only in stability, but also in absorption and release of the active ingredients. Some of these documents are the following.

Mexican patent application MX/a/2008/012643 of RECKITT BENCKISER HEALTHCARE (UK) LIMITED, published on 11/10/2007, claimed a medicament tablet containing acetaminophen as the (or an) active ingredient in an amount of between 200 to 800 mg, an encapsulated flavoring, and a second analgesic medicament selected from a group including, but not limited to, celecoxib, as well as a direct tableting method for manufacturing said tablet.

Another relevant document cited in MX/a/2008/012643 is the US Patent No. 4562024, which discloses an improved wet granulation process for preparing compressed tablets, particularly those having "an unsatisfactorily compressible drug; for example, acetaminophen." Therefore, it is clear that acetaminophen is a difficult material to tablet.

Also, the Mexican patent application PA/a/2004/007536 of PHARMACIA CORPORATION, published on 08/14/2003, claimed the use of a selective inhibitor of cyclooxygenase-2 or prodrug thereof, which is selected from a group that includes, but is not limited to celecoxib, and one or more active ingredients for colds or coughs, which includes, but is not limited to acetaminophen.

Then, the American patent application US200509051, published on 04/28/2005, whose owner is the company NORRIS MICHAEL C, claimed a pharmaceutical composition comprising a combination of a selective or specific inhibitor of COX-2 or a pharmaceutically acceptable salt or derivative thereof, an opiate or a pharmaceutically acceptable salt or derivative thereof, and a centrally acting cyclooxygenase inhibitor or a pharmaceutically acceptable salt or derivative thereof, as active ingredients, and a pharmaceutically acceptable carrier.

Wherein the selective or specific COX-2 inhibitor is selected from the group consisting of meloxicam, celecoxib, rofecoxib and pharmaceutically acceptable salts or derivatives thereof, and the centrally acting cyclooxygenase inhibitor is acetaminophen. The composition is for use in the treatment of pain with or without fever. On the other hand, there are some patent documents in the prior art that are of greater relevance, because they solve certain technical problems related to the celecoxib/acetaminophen combination:

For example, international patent application publication number WO2006043025 by BOOTS HEALTHCARE INT LTD, published on 27/04/2006, claimed a granular composition comprising a plurality of solidified molten granules including, but not limited to, a COX-2 drug such as celecoxib, and an additional pharmacologically active ingredient comprising ibuprofen or acetaminophen.

It also protects a process for producing said granular composition comprising the steps of: (a) forming a melt by melting a Cox-2 drug optionally in the presence of one or more excipients; and (b) forming the melt into a plurality of solidified molten granules.

In the specification of WO2006043025 it is said that the formulations prepared from the granular composition are stable during storage and have advantageous dissolution properties. It also mentions that the granular composition typically exhibits desirable flow and compressibility characteristics and can be compressed using minimal excipients without sticking or caking during the tableting process to provide a dosage form having suitable hardness properties combined with advantageous disintegration properties. However, no examples demonstrating such stability during storage are provided in the application.

Even, as it will be understood later, the process disclosed in WO2006043025 differs from what is claimed in the present document, firstly, because the application of BOOTS HEALTHCARE INT LTD involves an initial stage that is carried out in a melt extruder, where a mass is obtained by completely melting a Cox-2 drug. In the specification of said application it is said that in a preferred embodiment the Cox-2 drug, and any additional components are fed to an extruder type system (preferably after having been combined). The materials are heated and mixed in the extruder until the Cox-2 drug is completely melted and a uniform mixture is produced. The Cox-2 drug, and any additional components are extruded and the extrudate is cooled. Preferably, the Cox-2 drug, and any additional components are extruded in a twin-screw extruder. The hot mass (comprising the Cox-2 drug and any additional components) extruded forms an agglomerated mass that can be collected and, if desired, ground to form granules. It is immediately noticeable that such procedures present in WO2006043025 will increase the cost and complexity of the overall manufacturing process. Which is not efficient and therefore not desirable. Furthermore, there are other drawbacks to the preferred process as described in WO2006043025; for example, the extruder is provided with a cooling zone, necessary to remove the heat generated by the kneading action on the material being extruded. In the cooling zone, the internal heat generated within the material being extruded is carried away so that partial melting of the Cox-2 drug cannot occur, which can be detrimental to the performance of the material in the extruder. This requires temperature controls, making the manufacturing process more complex and, consequently, unnecessary variables are added that could impact the final performance of the finished product. Another deficiency of the process disclosed in WO2006043025 occurs when the same application mentions that the Cox-2 drug is substantially completely melted, since a liquid is formed, and the additional components used are often insoluble in the melt of the Cox-2 drug producing a dispersion of the solid additional component within the liquid melt. This requires an extra mixing process so that the additional drug is homogeneously combined with the molten COX-2 drug. Thus, it can be again observed that the process claimed in WO2006043025 can be improved and optimized so that a stable product is truly obtained, and consequently, reduce the variables that impact the performance and quality of the final medicament, in addition to promoting lower production costs.

Consecutively, Mexican patent application MX/a/2011/011928, owned by BAYER HEALTHCARE LLC and published on 18/11/2010, claimed a mixture to be formed and provide a pharmaceutical compressed tablet, characterized in that it comprises crystals of a medicinally active ingredient having a particle size of less than about 40 µm and a dissolution aid, wherein said mixture has been sufficiently ground to substantially coat the crystals with the dissolution aid, whereby the dissolution rate of the medicinally active ingredient when formed into a compressed tablet is enhanced compared to the dissolution rate of said active ingredient in a compressed tablet without such grinding. The mixture is characterized in that the medicinally active ingredient is selected from among other compounds, celecoxib, acetaminophen, and mixtures thereof; and the dissolution aid is selected from the group consisting of sodium and calcium salts of carbonates and bicarbonates. Although the main objective of this patent application is to provide a technique to increase the solubility of pharmaceutical specialties in compressed tablet form with the purpose of decreasing the residence time in the stomach, it does not solve the technological challenge of providing improved stability for the celecoxib/acetaminophen combination.

Consecutively, in Mexican patent MX 202128 of EDWARD MENDELL CO. INC., it is mentioned that acetaminophen is considered a high-load active ingredient because most commercial compressed tablet formulations include 70% to 85% by weight of acetaminophen per finished tablet. This high active ingredient loading combined with the practically poor physical characteristics for direct compression have not allowed pharmaceutical formulators to use direct compression techniques to prepare the final tablets. Therefore, a limitation of direct compression as a method for tablet production is the potential size of the compressed tablet. If the amount of active ingredient is high, the pharmaceutical formulator may select wet granulation with other excipients to achieve the acceptable tablet size with the desired amount of acetaminophen.

Currently many experts in the art prefer wet granulation to direct compression because wet granulation has a greater likelihood of overcoming any problems associated with the physical characteristics of the different ingredients in the formulation, therefore a material having the flow and cohesion characteristics that are necessary requirements for obtaining an acceptable solid dosage form is desirable. Other advantages of wet granulation are first, that through this process the material to be compressed is obtained with better moisture properties, particularly in the case of hydrophobic medicament substances. The addition of a hydrophilic excipient makes the surface of a hydrophobic drug more hydrophilic, facilitating disintegration and dissolution. Secondly, the uniform content of solid dosage forms is generally improved with the wet granulation method because all the granules obtained therefore usually contain approximately the same amount of medicament. This prevents segregation of the different ingredients of the material to be compressed (due to different physical characteristics). Finally, the particle size and shape of the particles comprising the granulate to be compressed are optimized by the wet granulation process. This is because when the dry solid is granulated with moisture the binder "glues" the particles together in such a way that they clump together into granules that are more or less spherical.

As it can be seen, patent MX202128 applies wet granulation techniques for the active ingredient acetaminophen, however, none of the documents in the state of the art is aimed at optimizing the stability of the compressed tablets of the combination of celecoxib with acetaminophen during storage, in a way that allows maintaining and ensuring the chemical characteristics of each active ingredient in the medicament.

Given these facts, there is a need to find a process that solves the technical problem of the high adhesiveness and low solubility of celecoxib, which, when combined with acetaminophen, a drug that has low compaction, could delay the dissolution of the latter and therefore affect its stability. The technological complexity lies in the combination of two drugs with different doses and rheological, compaction and solubility characteristics; on the one hand, celecoxib is a non-steroidal anti-inflammatory drug, liposoluble, with poor flow and excessive adhesiveness and on the other, acetaminophen is an antipyretic and analgesic drug, water-soluble, with poor flow and low compaction; therefore, there is a need for a combination of both drugs that is stable under the influence of environmental factors such as temperature, humidity or light in the packaging that contains it, with an effective absorption and release mechanism, which allows analgesia and a reduction in adverse effects, also demonstrating no pharmacokinetic interaction compared to monodrugs.

### SUMMARY OF THE INVENTION

The main object of the invention is to provide a pharmaceutical composition comprising celecoxib and acetaminophen in form of an immediate release coated tablet, as well as an optimized process for its manufacture. The composition and the process avoid the chemical interactions existing when combining celecoxib with acetaminophen and solve the technical problem that involves the combination of two drugs with different doses and rheological characteristics of compaction and solubility; so that the stability of the product is guaranteed.

The process for manufacturing the stable pharmaceutical composition of celecoxib with acetaminophen comprises the following steps:
a) mixing the active ingredients celecoxib and acetaminophen with at least a humectant, a binder, a disintegrant, a glider and a diluent for approximately 7 minutes;
b) moistening with purified water and granulating the mixture of the previous step;
c) drying the granulate obtained in the previous step until reaching a humidity between approximately 1.8% and 2.0%;
d) adding a binder and a disintegrant, mixing for approximately 5 minutes;
e) moistening with purified water and performing a second granulation;
f) drying the granule obtained in the previous step until reaching a humidity between approximately 1.3% and 1.5%;
g) reducing the size of the granules;
h) adding a disintegrant, an alkalizing and a diluent, mixing for approximately 5 minutes;
i) adding a lubricant and mixing for approximately 3 minutes;
j) compressing the mixture obtained in the previous step into 1200 ± 60 mg cores;
k) coating the cores obtained in the previous with 2.0% coating.

In one embodiment wherein the pharmaceutical composition comprises a dosage of 200 mg of celecoxib and 500 mg of acetaminophen, the tablet cores have a weight of 1200 mg ± 60 mg.

In an embodiment wherein the pharmaceutical composition comprises a dosage of 200 mg of celecoxib and 200 mg of acetaminophen, the tablet cores have a weight of 600 mg ± 30 mg.

The improved stability pharmaceutical composition obtained by the process described above has a hardness of 6.0-13.0 Kp and a disintegration time of less than 9 minutes.

Another object of the invention is to provide a solid pharmaceutical composition with immediate release and improved stability for oral administration in a single dosage unit, consisting of the combination of two active ingredients: celecoxib and acetaminophen, intended for the treatment of pain, fever and minor ailments of different etiologies, resulting from applying the optimized process.

In one embodiment of the invention, a stable pharmaceutical composition with pharmaceutically acceptable excipients and a process for manufacturing the same are provided. Celecoxib is present in an amount of 200 mg with acetaminophen in an amount of 200 mg or 500 mg. The pharmaceutical composition contains, but is not limited to, excipients including surfactants or humectants, binders, diluents, disintegrants, glidants, alkalizing, lubricants, and coating agents.

In another embodiment of the invention, the composition is for oral administration.

In another additional embodiment of the invention, the pharmaceutical composition is formulated in a solid pharmaceutical form, preferably, it is formulated in the form of tablets.

### BRIEF DESCRIPTION OF THE FIGURES

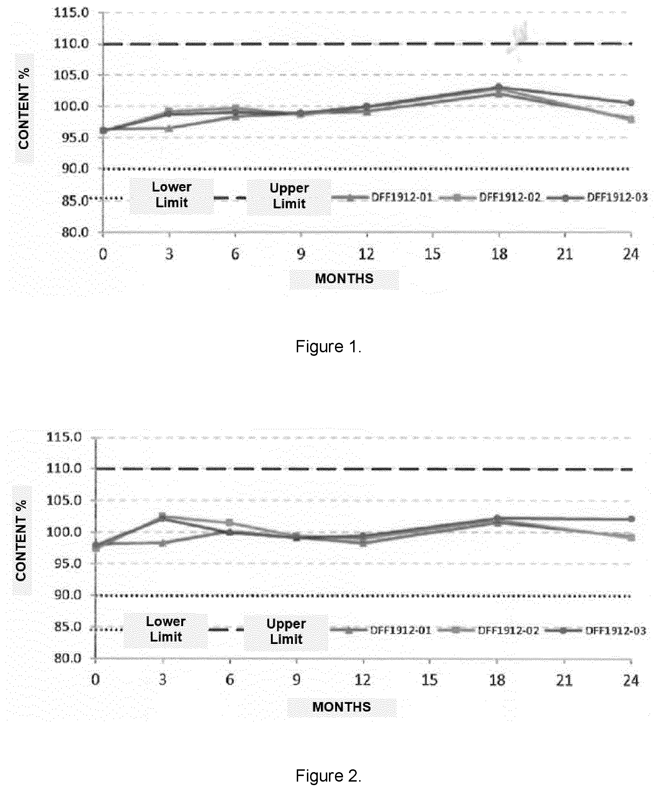

**Figure 1****.** Tendency of celecoxib content results for the product celecoxib-acetaminophen 200 mg/ 200 mg Tablets, batches DFF1912-01, DFF1912-02, and DFF1912-03, for 24 months under the Condition of 30 °C ± 2 °C/ 75% RH ± 5% RH.
**Figure 2****.** Tendency of acetaminophen content results for celecoxib-acetaminophen 200 mg/ 200 mg Tablets, batches DFF1912-01, DFF1912-02, and DFF1912-03, for 24 months under the Condition of 30 °C ± 2 °C/ 75% RH ± 5% RH.

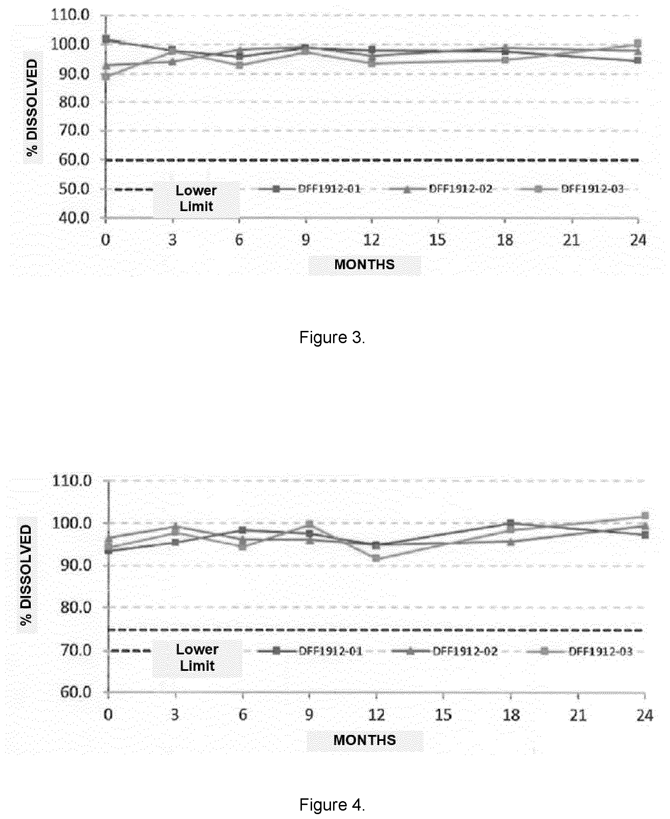

**Figure 3****.** Tendency of celecoxib dissolution results from the product celecoxib-acetaminophen 200 mg/200 mg Tablets, batches DFF1912-01, DFF1912-02, and DFF1912-03, for 24 months under the Condition of 30 °C ± 2 °C/75% RH ± 5% RH.
**Figure 4****.** Tendency of acetaminophen dissolution results for celecoxib-acetaminophen 200 mg/200 mg Tablets, batches DFF1912-01, DFF1912-02, and DFF1912-03, for 24 months under the Condition of 30 °C ± 2 °C/ 75% RHt 5% RH.

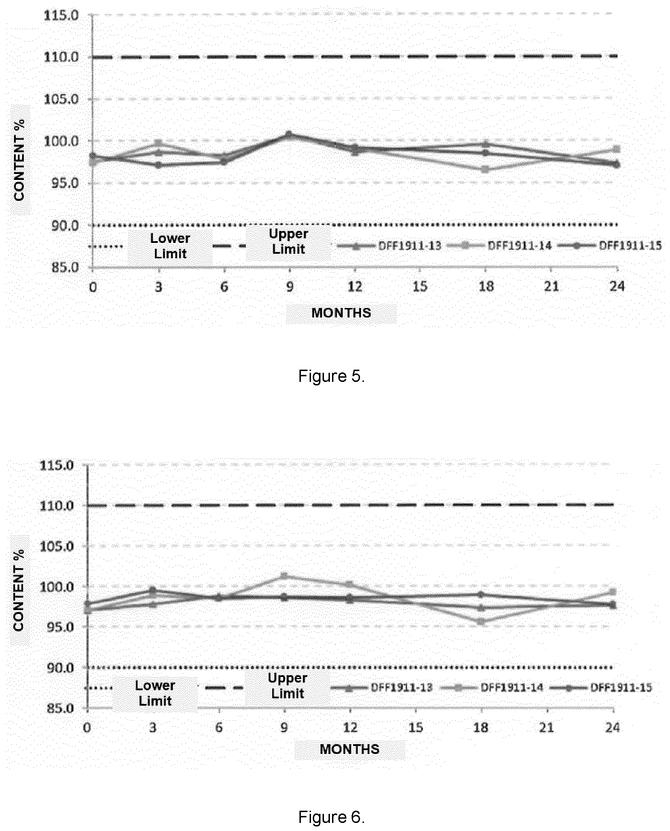

**Figure 5****.** Tendency of celecoxib content results for the product celecoxib-acetaminophen 200 mg/ 500 mg Tablets, batches DFF1911-13, DFF1911-14, and DFF1911-15, for 24 months under the Condition of 30 °C ± 2 °C/ 75% RH ± 5% RH.
**Figure 6****.** Tendency of acetaminophen content results for celecoxib-acetaminophen 200 mg/ 500 mg Tablets, batches DFF1911-13, DFF1911-14, and DFF1911-15, for 24 months under the Condition of 30 °C ± 2 °C/ 75% RH ± 5% RH.

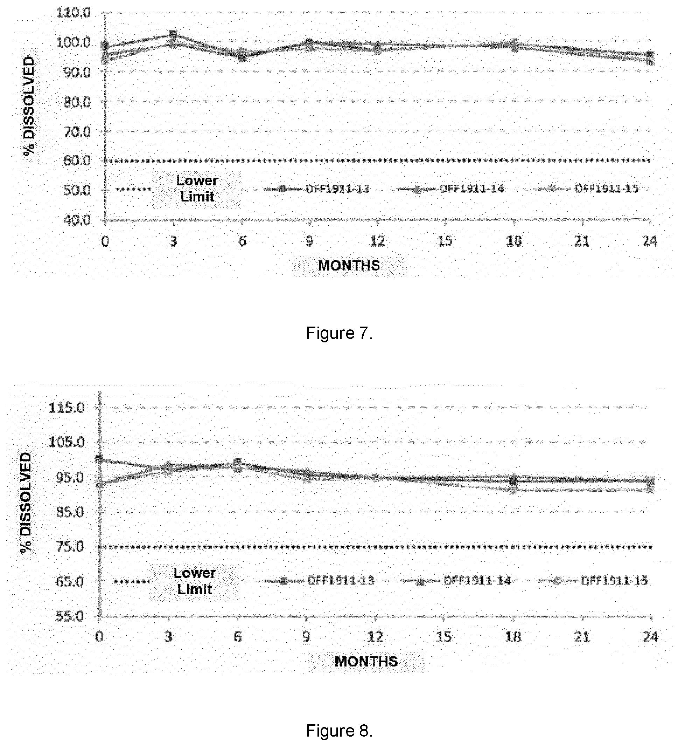

**Figure 7****.** Tendency of celecoxib dissolution results from the product celecoxib-acetaminophen 200 mg/500 mg Tablets, batches DFF1911-13, DFF1911-14, and DFF1911-15, for 24 months under the Condition of 30 °C ± 2 °C/ 75% RH ± 5% RH.
**Figure 8****.** Tendency of acetaminophen dissolution results for celecoxib-acetaminophen 200 mg/500 mg Tablets, batches DFF1911-13, DFF1911-14, and DFF1911-15, for 24 months under the Condition of 30 °C ± 2 °C/75 % RH ± 5% RH.

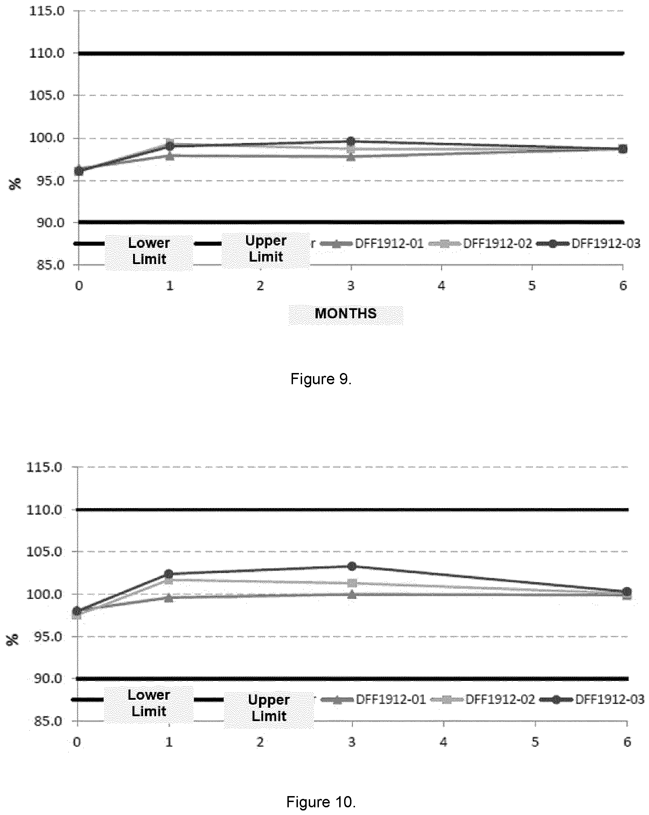

**Figure 9****.** Tendency of celecoxib content results for the product celecoxib-acetaminophen 200 mg/200 mg Tablets, batches DFF1912-01, DFF1912-02, and DFF1912-03, for 6 months under the Condition of 40 °C ± 2 °C/ 75% RH ± 5% RH.
**Figure 10****.** Tendency of acetaminophen content results for celecoxib-acetaminophen 200 mg/ 200 mg Tablets, batches DFF1912-01, DFF1912-02, and DFF1912-03, for 6 months under the Condition of 40 °C ± 2 °C/ 75% RH ± 5% RH.

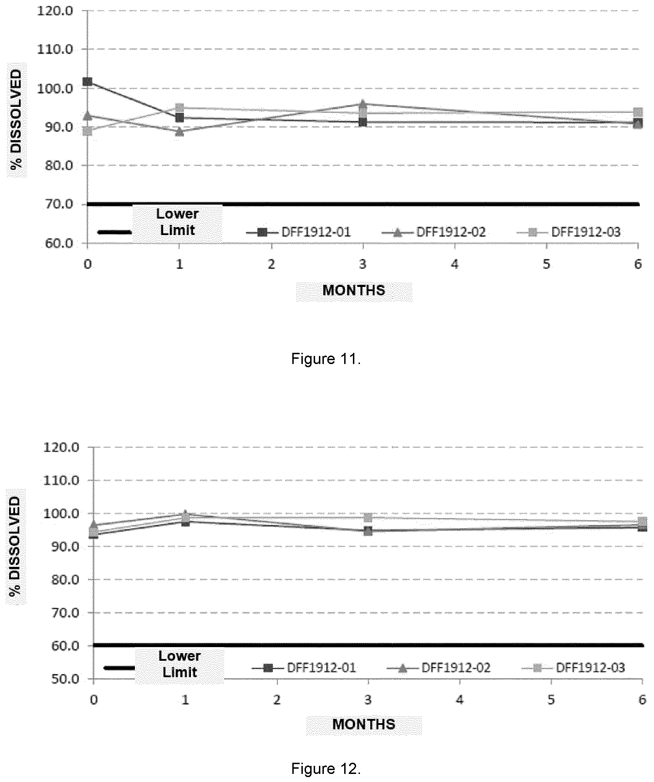

**Figure 11****.** Tendency of celecoxib dissolution results from the product celecoxib-acetaminophen 200 mg/200 mg Tablets, batches DFF1912-01, DFF1912-02, and DFF1912-03, for 6 months under the Condition of 40 °C ± 2 °C/ 75% RH ± 5% RH.
**Figure 12****.** Tendency of acetaminophen dissolution results for celecoxib-acetaminophen 200 mg/200 mg Tablets, batches DFF1912-01, DFF1912-02, and DFF1912-03, for 6 months under the Condition of 40 °C ± 2 °C/ 75% RH ± 5% RH.

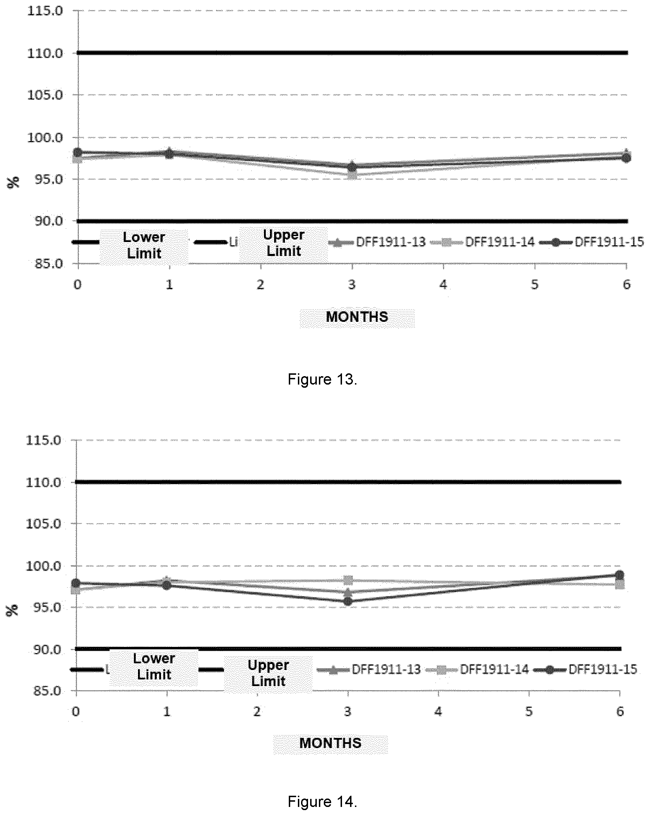

**Figure 13****.** Tendency of celecoxib content results for the product celecoxib-acetaminophen 200 mg/500 mg Tablets, batches DFF1911-13, DFF1911-14, and DFF1911-15, for 6 months under the Condition of 40 °C ± 2 °C/ 75% RH ± 5% RH.
**Figure 14****.** Tendency of acetaminophen content results for celecoxib-acetaminophen 200 mg/ 500 mg Tablets, batches DFF1911-13, DFF1911-14, and DFF1911-15, for 6 months under the Condition of 40 °C ± 2 °C/ 75% RH ± 5% RH.

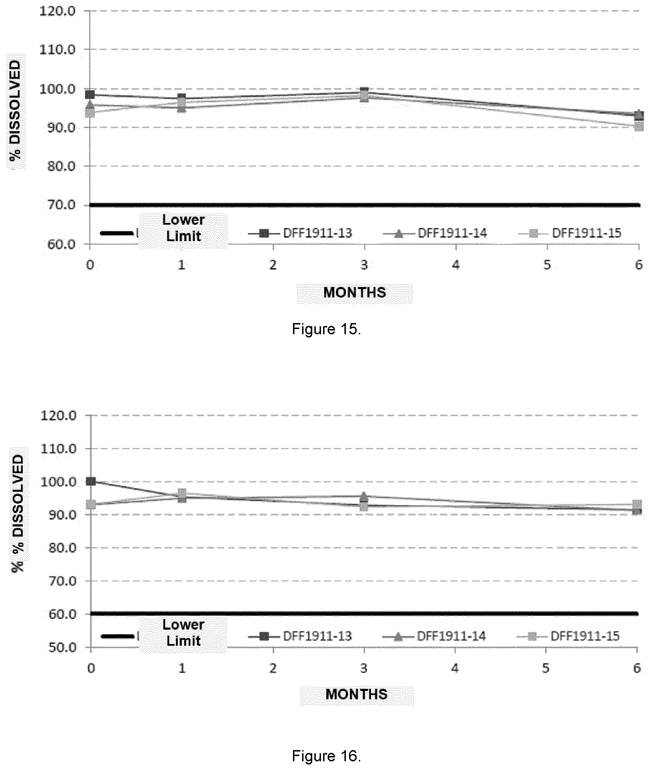

**Figure 15****.** Tendency of celecoxib dissolution results from the product celecoxib-acetaminophen 200 mg/500 mg Tablets, batches DFF1911-13, DFF1911-14, and DFF1911-15, for 6 months under the Condition of 40 °C ± 2 °C/ 75% RH ± 5% RH.
**Figure 16****.** Tendency of acetaminophen dissolution results of celecoxib-acetaminophen 200 mg/500 mg Tablets, batches DFF1911-13, DFF1911-14, and DFF1911-15, over 6 months under the condition of 40 °C ± 2 °C/ 75% RH ± 5% RH.
**Figure 17****.** Mean plasma concentrations and standard error for celecoxib.
**Figure 18****.** Log-transformed mean plasma concentrations and standard error of celecoxib. Original concentrations in µg/mL.
**Figure 19****.** Mean plasma concentrations and standard error for acetaminophen.
**Figure 20****.** Log-transformed mean and standard error of acetaminophen plasma concentrations. Original concentrations in µg/mL.

### DETAILED DESCRIPTION OF THE INVENTION

### Definitions:

The term "active ingredient" as used herein refers to the substance in a product that has biological activity.

The term "excipient" as used herein includes any substance used as a vehicle for the administration of the active ingredient to a subject, and any substance added to the active ingredient, for example, to improve its handling properties or to enable the resulting composition to be produced in a pharmaceutical form for oral administration having the desired shape and consistency. Excipients may include, by way of illustration and not limitation, diluents, disintegrants, binders, adhesives, humectants, lubricants, glidants, substances added to mask a bad taste, substances added to improve the appearance of a pharmaceutical form, and any other substance conventionally used in the preparation of oral pharmaceutical forms.

The term "dose unit" as used herein refers to an amount of a pharmaceutical composition intended for a single administration.

The term "oral administration pharmaceutical form": means a dosage unit of a pharmaceutical composition intended to be administered to the gastrointestinal tract of a subject through the mouth of said subject and for the purposes of the present invention.

The term "Shelf life": means the date until which the drug is guaranteed to be stable in its original packaging or container, unopened and unhandled and stored under appropriate conditions.

The term "adverse reaction" refers to an unwanted response to a medicament, in which the causal relationship with it is, at least, reasonably attributable.

The term "Dissolution profile" refers to the experimental determination of the amount of drug dissolved at various times, under controlled experimental conditions, from the pharmaceutical form.

The term "Improved Stability" refers to a scientific technical design that is conducted on a formulation for a certain time, under the influence of temperature, humidity or light in the packaging that contains it, to demonstrate the shelf-life period and determine its expiration date.

The term "Highly soluble active" refers to a drug with a water solubility of 50 to 100 % in a 1:1 m/s ratio.

Now, to improve the understanding of this invention, it is necessary to explain the following concepts.

### Stability

In the Mexican Official Standard NOM-073-SSA1-2015, "Stability of drugs and medications, as well as herbal remedies" the concept of stability is defined as the capacity of a drug, medicament or herbal remedy contained in a vessel-closure system of a certain material, to maintain, during the time of storage and use, the established quality specifications.

Stability studies are tests conducted on a drug or medicament for a certain period under the influence of temperature, humidity or light in the packaging that contains it, to demonstrate its shelf life and determine its expiration date.

These studies also allow for the assignment/confirmation of expiration/reanalysis periods, bulk storage times or intermediate products stored during the process, the establishment of storage and transport conditions, as well as the selection of the best vessel-closure system. For the Mexican Republic, the Climatic Zone recognized for conducting these stability studies is Climatic Zone II (subtropical, with possible high humidity), according to the classification of the World Health Organization.

Prior to the start of the stability study, a complete analysis of the drug must be conducted; through tests that include general analysis methods such as:
*Dose uniformity.* It is demonstrated through the Content Uniformity method, which is based on the quantitative determination of the individual content of the active ingredient in a certain number of units of single-dose pharmaceutical forms to determine whether the variation of the individual contents expressed in terms of relative standard deviation is within the established limits.
*Dissolution.* This method is used to determine the release of an active principle into the test medium, from the dosage form that contains it. The test involves a series of variables of diverse origin that affect the hydrodynamic flow pattern at the solid-liquid interface, which, in turn, is a determining factor in the dissolution rate and in obtaining reproducible test results.
*Related substances.* These are drug-like compounds that can be generated during the drug production process.

The tests conducted in the stability studies of the present invention are defined in NOM-073-SSA1-2015 as follows:
The conditions of the stability study and its duration must be sufficient to cover the storage, distribution and use of the drug, and in accordance with section 10.9 of NOM-073-SSA1-2015, "When the long-term stability study of at least 3 commercial batches demonstrates a period equal to or greater than 24 months, the expiration period corresponding to the supported shelf life will be granted."

Long-term stability studies: those designed under temperature and humidity storage conditions, which allow testing the storage and shelf-life conditions of a drug, medicament, or herbal remedy, through a program of sampling times and evaluation of the physical, chemical, and biological requirements, which verify the conservation of its properties during its shelf life.

*Accelerated stability studies*: They are designed under extreme storage conditions to increase the speed of chemical or biological degradation or physical changes of a drug, medicament, or herbal remedy.

The main object of the invention is to provide an optimized process that avoids the chemical interactions existing when combining celecoxib with acetaminophen for oral administration in a single dosage unit, and to solve the technical problem involved in combining two drugs with different doses and rheological characteristics of compaction and solubility; so that the stability of an immediate release product intended for the treatment of pain, fever and minor discomfort of different etiologies is guaranteed.

By improving product stability, the shelf life of the medicament is increased, and adverse reactions are reduced, as demonstrated in the Phase I clinical study that presents the incidence of adverse effects.

Consequently, this invention provides a manufacturing process in which, surprisingly, the selection of unit operations, the order and the execution time of each stage, are essential to control the different physicochemical properties of the drugs, ensuring their long-term conservation stability and their dissolution, so that it complies with the established quality characteristics, guaranteeing the identity, purity and potency of the product, so as to ensure its suitability for use.

To achieve this, a series of tests were conducted considering a wet granulation process, finding that, to obtain a stable product, under the required specifications, the critical operations are granulation time, mixing, compression and coating. Therefore, unit operations and excipients were selected according to the product behavior and requirements.

The variables that improved for celecoxib include its rheological and adhesive characteristics, and for acetaminophen the rheological and compaction characteristics were improved, ensuring the proper functioning of the product.

The pharmaceutical form selected for dosing the drugs was tablets due to their dosage accuracy. They can also be designed in the form of a caplet because they are easier to swallow and are the smallest possible size for the quantity and weight of the drugs.

According to another aspect of the invention, the stability test is conducted for a period of 6 months under the accelerated condition 40 °C ± 2 °C/ 75% ± 5% RH according to NOM-73.

The invention contemplates the stability attributes of the dosage form to remain compatible for a period equal to or greater than 24 months. The stability attributes of the pharmaceutical composition of the present invention are also evaluated under temperature and humidity test conditions in the long-term condition 30 °C ± 2 °C/ 75 % ± 5% RH to establish the shelf-life period.

### Qualitative - quantitative formulation

The pharmaceutical composition of the present invention comprises celecoxib and acetaminophen as active ingredients, wherein the pharmaceutical composition is an immediate release coated tablet.

In a preferred embodiment of the invention, the pharmaceutical composition comprising celecoxib and acetaminophen as active principles further comprises at least one of a surfactant/humectant agent, a binder, a diluent, a disintegrant, a slip, an alkalizing, a lubricant, a coating and a vehicle.

In another preferred embodiment, the clecoxib is in an amount equivalent to a dosage of 200 mg while the acetaminophen is in an amount equivalent to a dosage of 200 mg or 500 mg.

### Formulation components

The components of the formulation were selected based on their function; the absence or modification of one of them does not comply with the established quality characteristics. These components are:

| Drug 1/Name | Celecoxib |
|---|---|
| Chemical Structure | 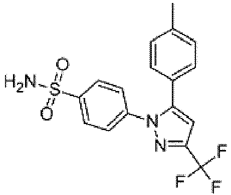 |
| Condensed formula | C₁₇H₁₄F₃N₃O₂S |
| Molecular weight | 381.375 g/mol |
| Description | White, odorless and tasteless crystals |
| Solubility | Very soluble in alcohol, methanol, acetone and chloroform; slightly soluble in ethyl acetate; practically insoluble in water. |
| Biopharmaceutical classification | Belongs to group II (high permeability and low solubility). |
| pKa | 11.1 |
| Melting point | 157-158 °C |
| Storage conditions | Store in a dry place at a temperature not exceeding 30 °C. |

| Drug 2/Name | Acetaminophen |
|---|---|
| | 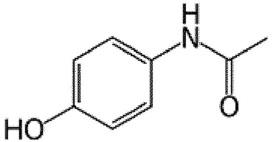 |
| Chemical Structure | C₈H₉NO₂ |
| Condensed formula | 151.17 g/mol |
| Molecular weight | White crystalline powder, odorless, with a slight bitter taste. |
| Description | |
| Solubility | Easily soluble in alcohol, soluble in boiling water and in 1 N sodium hydroxide. |
| Biopharmaceutical classification | Group III (high solubility and low permeability) |
| pKa | 9.38 |
| Melting point | 169-171 °C |
| Storage conditions | Protect this material from exposure to light. Keep away from oxidizing materials and store at room temperature. |

| Sodium lauryl sulfate | |
|---|---|
| A selected humectant at a concentration of 1.30%, an anionic surfactant that allows the insoluble drug to moisten, breaking the surface tension and achieving a solubilizing effect. | 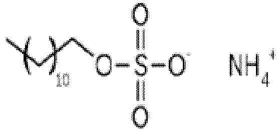 |
| Use interval: 1 to 25%. | |
| Other examples: Poloxamers, polyoxyethylene castor oil derivatives, benzalkonium chloride, benzethonium chloride, polyoxyethylene alkyl alkyl ethers, and polyoxyethylene sorbitan fatty acid esters. | |

| Polyvidone K30 | |
|---|---|
| A binder selected at a concentration of 2.16%, an additive with high binding power, which, by absorbing water during manufacturing, promotes granulation of the formulation components by forming bonds between the particles of the materials, improving the physical properties of the powder mixture, such as compaction, particle size, density and flow. | 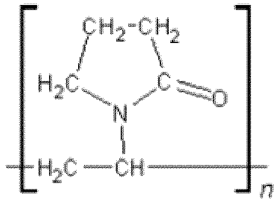 |
| Use interval: 1 to 25%. | |
| Other examples: Polyvidones in all K grades and their derivatives, cellulose derivatives such as carbomers, hydroxypropylcelluloses, carboxymethylcelluloses, starch derivatives such as pregelatinized starch and corn starch. | |

| Microcrystalline cellulose PH 101 | |
|---|---|
| A diluent selected for its properties at a concentration of 24.21% to obtain a mixture with good compaction and flow characteristics. | 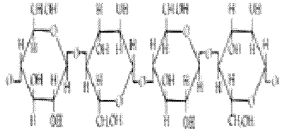 |
| Use interval: 5 to 90%. | |
| Other examples: Cellulose derivatives such as microcrystalline cellulose, phosphate derivatives such as dibasic calcium phosphate, starch derivatives such as pregelatinized starch and corn starch. | |

| | |
|---|---|
| Croscarmellose sodium | 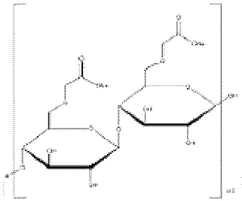 |
| A disintegrant selected due to its fibrous nature at a concentration of 4.08%, as it provides excellent water absorption possibilities, and its cross-linked chemical structure creates an insoluble hydrophilic product and high swelling properties. | |
| Use interval: 0.5 to 15%. | |
| Other examples: Cellulose derivatives such as hydroxypropylcelluloses, carboxymethylcelluloses, microcrystalline cellulose, povidone derivatives such as crospovidone, plasdons, starch derivatives such as pregelatinized starch, sodium starch glycolate, and corn starch. | |

| | |
|---|---|
| Colloidal Silicon Dioxide | |
| A slip agent that, at a concentration of 0.41%, improves the flow characteristics of the granules by reducing the friction between the particles, minimizing the formation of agglomerates. Its mechanism of action by dispersion of the electrostatic charges formed on the surface of the granules and reduction of the Van Der Waals forces to achieve the separation of the granules. | SiO₂ |
| Use interval: 1 to 2%. | |
| Other examples: Cellulose derivatives such as carboxymethyl celluloses, microcrystalline cellulose, starch derivatives such as pregelatinized starch and corn starch, silicate derivatives, e.g. magnesium silicate, magnesium trisilicate, and powdered talc. | |

| | |
|---|---|
| Sodium bicarbonate | |
| An alkalinizing agent suitable to increase the bioavailability of the drug in the body at a concentration of 8.17%. The incorporation of the bicarbonate generates an alkaline microenvironment in the tablet which, upon contact with the different media of the gastrointestinal tract, modifies the pH ensuring the adequate dissolution of celecoxib and, consequently, its absorption guaranteeing its bioavailability. | 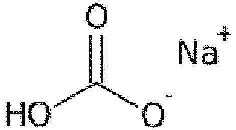 |
| Use interval: 10 to 50%. | |
| Other examples: Sodium carbonate, calcium hydroxide, potassium bicarbonate, potassium citrate, potassium hydroxide, sodium citrate dihydrate, and sodium hydroxide. | |

| | |
|---|---|
| Magnesium stearate | |
| It is a lubricant agent which, due to the acetaminophen adhesiveness characteristics, its addition is necessary in a concentration of 0.41% to avoid sticking between the different tooling during the compression process. | 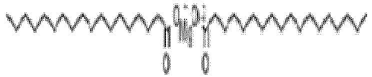 |
| Use interval: 0.25 to 10%. | |
| Other examples: Stearate derivatives such as zinc stearate, calcium stearate, stearic acid, monostearates, stearyl fumarate, sulfated derivatives such as magnesium lauryl sulfate, and magnesium lauryl sulphate; Talc. | |

| | |
|---|---|
| White Odapry YS-1-7003 | |
| It is a coating system based on the polymer hydroxypropylmethylcellulose which, at a concentration of 2.00%, provides protection and color to the combination. | 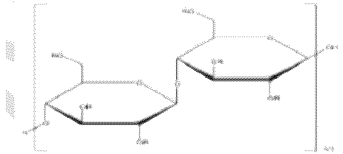 |
| Use interval: 0.5 to 6%. | |
| Other examples: Cellulose derivatives such as hydroxypropylmethylcellulose, hydroxypropylcellulose, carboxymethylcellulose, polyvinyl derivatives, such as polyvinyl alcohol; polyethylene glycol, povidones in all K grades, and their derivatives. | |

In another more preferred embodiment, the pharmaceutical composition comprising celecoxib and acetaminophen as active ingredients further comprises:
- sodium lauryl sulfate as surfactant/humectant agent;
- polyvidone as binder;
- microcrystalline cellulose as diluent;
- croscarmellose sodium as disintegrant;
- colloidal silicon dioxide as slip;
- sodium bicarbonate as alkalizing;
- magnesium stearate as a lubricant,
- a hydroxypropyl methylcellulose coating system;
- purified water as a vehicle.

In a preferred embodiment of the invention the technological development comprises the qualitative and quantitative formulation described below:

| mg/Tab | Components | Function |
|---|---|---|
| 200.00 | Celecoxib | Drug |
| 500.00 | Acetaminophen | Drug |
| 17.00 | Sodium lauryl sulfate | Surfactant/humectant |
| 26.50 | Polyvidone K30 | Binder |
| 296.50 | Microcrystalline cellulose PH101 | Diluent |
| 50.00 | Croscarmellose sodium | Disintegrant |
| 5.00 | Colloidal silicon dioxide | Slip |
| 100.00 | Sodium bicarbonate | Alkalinizing |
| 5.00 | Magnesium stearate | Lubricant |
| 24.50 | Opadry white YS-1-7003 | Protection and color |
| 0.635 | Purified water (mL) | Vehicle |

In another embodiment, the formulation consists of:

| mg/Tab | Components | Function |
|---|---|---|
| 200.00 | Celecoxib | Drug |
| 200.00 | Acetaminophen* | Drug |
| 11.45 | Sodium lauryl sulfate | Surfactant/humectant |
| 10.60 | Polyvidone K30 | Binder |
| 105.00 | Microcrystalline cellulose PH101 | Diluent |
| 28.50 | Croscarmellose sodium | Disintegrant |
| 2.00 | Colloidal silicon dioxide | Slider |
| 56.85 | Sodium bicarbonate | Alkalinizing |
| 2.00 | Magnesium stearate | Lubricant |
| 12.25 | Opadry white YS-1-7003 | Protection and color |
| 0.318 | Purified water (mL) | Vehicle |

Regarding the coating system, in a preferred embodiment it is Opadry white YS-1-7003, which is a coating system based on the polymer hydroxypropylmethylcellulose.

This formulation, for the manufacture of a drug, is useful in the treatment of pain, preferably acute pain and postoperative pain.

The formulations are prepared in a non-limiting manner according to the following process.

### Optimized manufacturing process.

The technological complexity lies in the combination of two drugs with different doses and rheological, compaction and solubility characteristics; on the one hand, celecoxib is a non-steroidal anti-inflammatory drug, liposoluble, with poor flow and excessive adhesiveness and on the other, acetaminophen is an antipyretic and analgesic drug, water-soluble, with poor flow and low compaction; therefore, there is a need for a combination of both drugs that is stable under the influence of environmental factors such as temperature, humidity or light in the packaging that contains it, with an effective absorption and release mechanism, which allows analgesia and a decrease in adverse effects, also demonstrating no pharmacokinetic interaction compared to monodrugs.

The process comprises the steps of:
a) mixing the active ingredients celecoxib and acetaminophen with at least a humectant, a binder, a disintegrant, a glider and a diluent for approximately 7 minutes;
b) moistening with purified water and granulating the mixture of the previous step;
c) drying the granulate obtained in the previous step until reaching a humidity between approximately 1.8% and 2.0%;
d) adding a binder and a disintegrant, mixing for approximately 5 minutes;
e) moistening with purified water and performing a second granulation;
f) drying the granule obtained in the previous step until reaching a humidity between approximately 1.3% and 1.5%;
g) reducing the size of the granules using 0.050-inch (0.127 cm) mesh;
h) adding a disintegrant, an alkalizing and a diluent, mixing for approximately 5 minutes;
i) adding a lubricant and mixing for approximately 3 minutes;
j) compressing the mixture obtained in the previous step cores;
k) coating the cores obtained in the previous with 2.0% coating.

In one embodiment wherein the pharmaceutical composition comprises a dosage of 200 mg of celecoxib and 500 mg of acetaminophen, the tablet cores have a weight of 1200 mg ± 60 mg.

In an embodiment wherein the pharmaceutical composition comprises a dosage of 200 mg of celecoxib and 200 mg of acetaminophen, the tablet cores have a weight of 600 mg ± 30 mg.

In a preferred embodiment, the humectant is sodium lauryl sulfate, the binder is polyvidone, the diluent is microcrystalline cellulose, the disintegrant is croscarmellose sodium, the slip is colloidal silicon dioxide, the alkalizing is sodium bicarbonate, the lubricant is magnesium stearate, and the coating system is hydroxypropyl methylcellulose.

In a still more preferred embodiment, the process for manufacturing the pharmaceutical composition in accordance with the present invention comprises the following steps:
1. A sifting operation is performed by mesh of 0.157 cm (0.062 inches), to the following materials celecoxib, acetaminophen as active substances and the following materials: sodium lauryl sulfate (humectant), 50% polyvidone K30 (binder), 37.50% croscarmellose (disintegrant), colloidal silicon dioxide (slip) and 30.0 % microcrystalline cellulose PH 101.
2. The sieve from step No. 1 is added to a diffusion mixer and mixed for 7 minutes.
3. The mixture from step No. 2 is added to a granulator; it is moistened and granulated with purified water.
4. In the granulator equipment, the granule obtained from step No. 3 is dried until reaching a humidity between 1.8% and 2.0%.
5. The granules from step No. 4, 50 % of polyvidone K 30 and 37.5% of croscarmellose sodium are added.
6. The added materials from step No. 5 are mixed with the granules from step No. 4 for 5 minutes.
7. A second granulation is performed on the mixture from step No. 6; it is moistened and granulated with purified water.
8. In the granulator equipment, the granule obtained from step No. 7 is dried until reaching a humidity between 1.3% and 1.5%.
9. A size reduction operation, preferable using 0.050-inch mesh, is performed on the granules obtained in step No. 8.
10. A sifting operation is conducted, preferable using a 0.157 cm (0.062-inch) mesh, adding 25% croscarmellose sodium (disintegrant), sodium bicarbonate (alkalizing) and 70% microcrystalline cellulose PH101 (diluent).
11. The sieves from steps No. 9 and 10 are added to a diffusion mixer for 5 minutes.
12. A sifting operation is performed, preferable through a 0.157 cm (0.062-inch) mesh, and magnesium stearate (lubricant) is added.
13. The mixture from step No. 11 and the sieving from step No. 12 are added to a diffusion mixer for 3 minutes.
14. The final powder mixture is compressed into cores.
15. The cores obtained from step No. 9 are given a 2.0 % coating.

In a preferred embodiment, the granulation is wet granulation.

In one embodiment wherein the pharmaceutical composition comprises a dosage of 200 mg of celecoxib and 500 mg of acetaminophen, the tablet cores have a weight of 1200 mg ± 60 mg.

In an embodiment wherein the pharmaceutical composition comprises a dosage of 200 mg of celecoxib and 200 mg of acetaminophen, the tablet cores have a weight of 600 mg ± 30 mg.

In a preferred embodiment, the improved stability pharmaceutical composition obtained by the process described above has a hardness of 6.0-13.0 Kp and disintegration time of less than 9 minutes.

The manufacturing was conducted considering the physicochemical characteristics of the drugs; for celecoxib, its rheological characteristics and adhesiveness were improved and for acetaminophen its rheological and compaction characteristics were improved, ensuring its stability and dissolution.
in a preferred embodiment, the granulation is wet granulation, which allows obtaining the granules in a cohesive mixture, optionally with one or more excipients.

On the other hand, the mixing stages ensure the homogeneous distribution of the drugs with the excipients of the formulation, solubility is promoted, and the conformation of the granules is improved; the bioavailability of the drugs is also improved.

Regarding the coating, it is common knowledge that increases greater than 1 % in coating allow protection from bitter flavors and provide mechanical resistance, improve dissolution, mechanical resistance and generally improve the organoleptic properties of the product.

As a result of the optimized process, a product is obtained that combines the drugs celecoxib and acetaminophen in a single dosage unit, preferably with doses of [200 mg/500 mg], and [200 mg/200 mg], which represents a set of important technological challenges due to the physicochemical properties of each drug and the difference in doses of the combination. The present invention guarantees the obtaining of a stable product, where the combination of active ingredients does not present pharmacokinetic interaction compared to each drug separately.

Stability was achieved when the two active substances were joined together using solid bridges, taking advantage of the advantages and disadvantages of each Active Pharmaceutical Ingredient (API). For example, celecoxib is a water-insoluble and adhesive drug, so the addition of a highly soluble (acetaminophen) and non-adhesive active substance improves the rheological and dissolution properties; on the contrary, acetaminophen is a highly soluble and non-compactable active substance, so the addition of solid bridges with the insoluble active substance (celecoxib) will improve the rheological and compactability properties. On the other hand, the action of the alkalizing agent is suitable for increasing the bioavailability of the drug in the body. The incorporation of the alkalizing agent generates an alkaline microenvironment in the tablet that, upon contact with the different media of the gastrointestinal tract, modifies the pH, ensuring the proper dissolution of celecoxib and consequently its absorption, guaranteeing its bioavailability.

In accordance with yet another aspect of the present invention, the various stability attributes assessed for the tablet composition include the dissolution profile of the disintegration time.

Once the reasons why, by applying the process of the present invention, a stable product would be obtained have been explained, an example of an inadequate manufacturing process is provided below which, certainly, does not allow obtaining a stable product.

### Improper manufacturing process.

### Example 1. Physical mixing.

1. A sifting operation, preferable using a 0.157 cm mesh (0.062 inches), was performed placing 200 mg of celecoxib, 500 mg of acetaminophen as active substances and the percentage of the following materials: 1.87% of a solubilizer, 5.40% of a diluent 1, 3.12% of a diluent 2, 5.0% of a disintegrant, 0.31% of a slip and 0.3% of an anti-adhesive.
2. The sieve from step No. 1 was added to a diffusion mixer for 5 minutes.
3. A sifting operation, preferable using a 0.157 cm mesh (0.062 inches), was performed, placing 0.61% of lubricant.
4. The mixture from step number 2 and the sieving from step No. 3 were added to a diffusion mixer for 3 minutes.
5. The mixture obtained from step No. 4 was processed in a rotary tablet press to equivalent weights between 810 and 890 mg per tablet.

### Example 2. Dry Granulation:

1. A sifting operation, preferable using a 0.157 cm mesh screening operation (0.062 inches) was performed placing 200 mg of celecoxib, 500 mg of acetaminophen as active substances and the percentage of the following materials: 1.71% of a solubilizer, 3.02% of a binder, 4.58% of a disintegrant, 0.29% of a slip, and 12.09% of a diluent.
2. The sieve from step No. 1 was added to a diffusion mixer, it was mixed for 5 minutes.
3. Dry granulation was performed by extrusion to the mixture from step No. 2.
4. A sifting operation was performed, preferable using a 0.127 cm mesh (0.050-inch mesh), on the granules obtained in step No. 3.
5. A sifting operation was conducted, preferable using a 0.257 cm mesh (0.062 inches), placing 0.42% of disintegrant, 5.2% of solubilizer and 15.0% of diluent.
6. The sieves from steps No. 4 and 5 were added to a diffusion mixer, it was mixed for 5 minutes.
7. A sifting operation, preferable using a 0.157 cm mesh (0.062 inches), was performed, placing 1.0% of lubricant.
8. The mixture from step No. 6 and the sieving from step No. 7 were added to a diffusion mixer, it was mixed for 3 min.
9. The mixture obtained from step No. 8 was processed in a rotary tablet press to equivalent weights between 950 mg and 1050 mg per tablet.

### Example 3. Wet granulation of each active substance:

1. A sifting operation, preferable using a 0.157 cm mesh (0.062 inches), was performed to homogenize the particle size of the drugs and materials, placing 200 mg of celecoxib and the percentage of the following materials: 0.97% of a humectant, 1.62% of a binder, 0.44% of a disintegrant, 0.17% of a slip and 2.81% of a diluent.
2. The sieve from step No. 1 was added to a diffusion mixer, it was mixed over 5 minutes.
3. A wet granulation of the powder mixture from step No. 2 was performed, obtaining a humidity of less than 0.7%.
4. A size reduction operation, preferable by 0.127 cm mesh (0.050 inches), was performed on the granules obtained in step No. 3.
5. A sifting operation was performed, preferable using a 0.157 cm mesh (0.062 inches), placing 500 mg of acetaminophen and the percentage of the following materials: 0.98% of a humectant, 1.63% of a binder, 0.43% of a disintegrant, 0.16% of a slip and 2.81% of a diluent.
6. A sifting operation was conducted, preferable using a 0.157 cm mesh (0.062 inches), placing 1.02% of a disintegrant, 4.08% of a solubilizer and 16.95% of a diluent.
7. The sieve from step No. 6 was added to a diffusion mixer, it was mixed over 5 minutes.
8. Wet granulation was performed on the powder mixture from step number 7, obtaining a humidity between 2.0% and 2.7%.
9. A sifting operation was performed, preferable through a 0.127 cm mesh (0.050 inches), on the granules obtained in step No. 8.
10. The sieves from step number 4 and step No. 9 were added to a diffusion mixer, it was mixed for 5 minutes.
11. A sifting operation was performed, preferable using a 0.157 cm mesh (0.062 inches), adding 0.33% of a lubricant.
12. The mixture from step No. 10 and the mixture from step No. 11 were added to a diffusion mixer, it was mixed for 3 minutes.
13. The mixture obtained from step No. 8 was processed in a rotary tablet press to equivalent weights between 760 and 840 mg per tablet.

The following unit operations do not give a stable product:
Physical mixing: The high adhesiveness of the celecoxib active ingredient causes problems in the rheological properties and, with respect to the acetaminophen active substance, its low compactability causes low mechanical resistance.

Dry granulation: Likewise, the high adhesiveness of the active substance celecoxib causes rheological problems with both active substances.

Wet Granulation (in an inadequate process): The two active substances are made separately (as monodrugs), then they are combined into a single mixture and compressed. This separate unit operation does not provide a stable product, in addition to not complying with the dissolution profiles of each asset.

Since the results have been compared with embodiments where the stability claimed in the present invention is not obtained; and once the invention has been described by means of preferred embodiments thereof, it must be understood that variations can be made thereto by a person with average knowledge in the art, without such modifications departing from the soul of the present invention. Therefore, such modifications are contemplated within the scope of the following claims:
***Long-Term Stability Study (30* °C ± *2 °C*/ *75% RH* ± *5% RH) for Celecoxib*/*Acetaminophen [200 mg*/*200 mg]***

Long-term stability results for batches DFF1912-01, DFF1912-02 and DFF1912-03 (30 °C ± 2 °C / 75% ± 5% RH) are shown below.

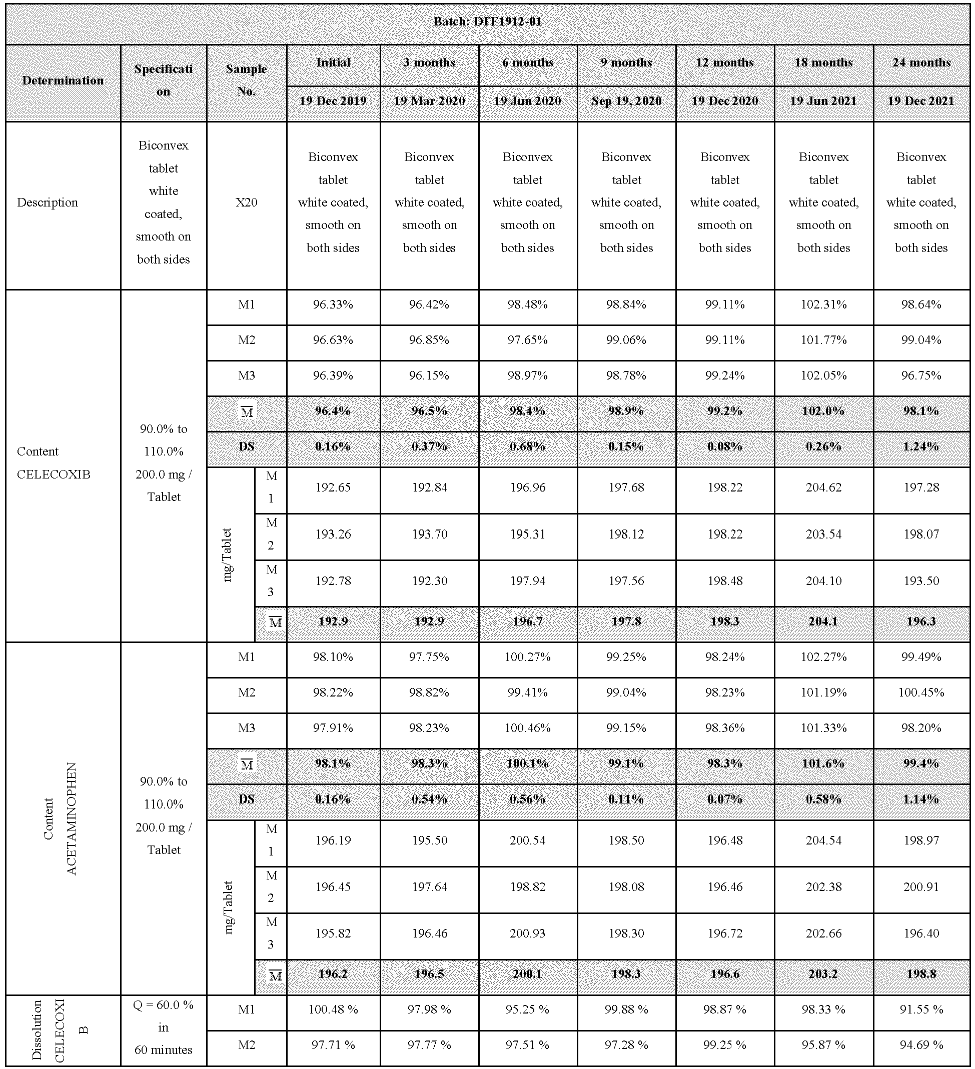

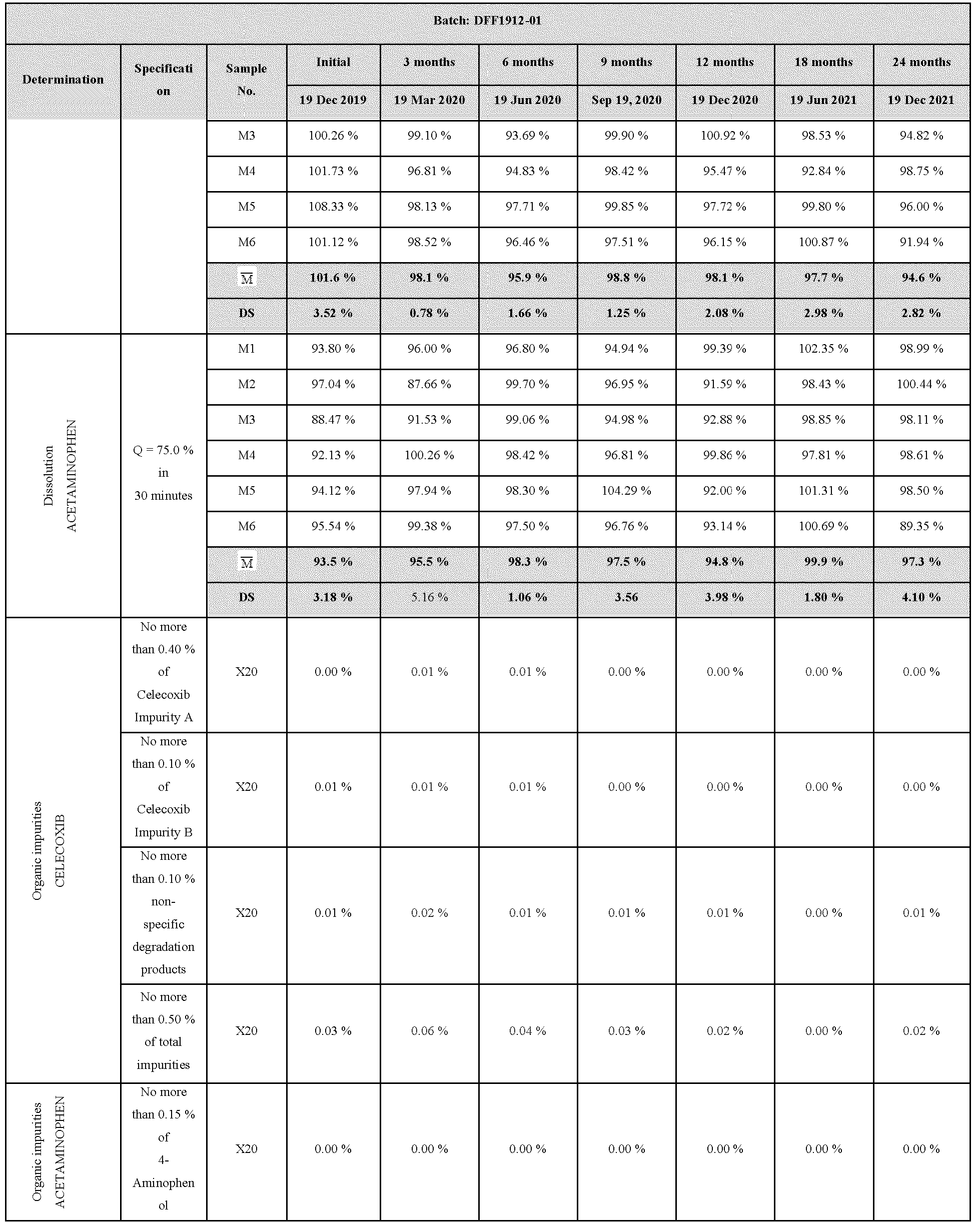

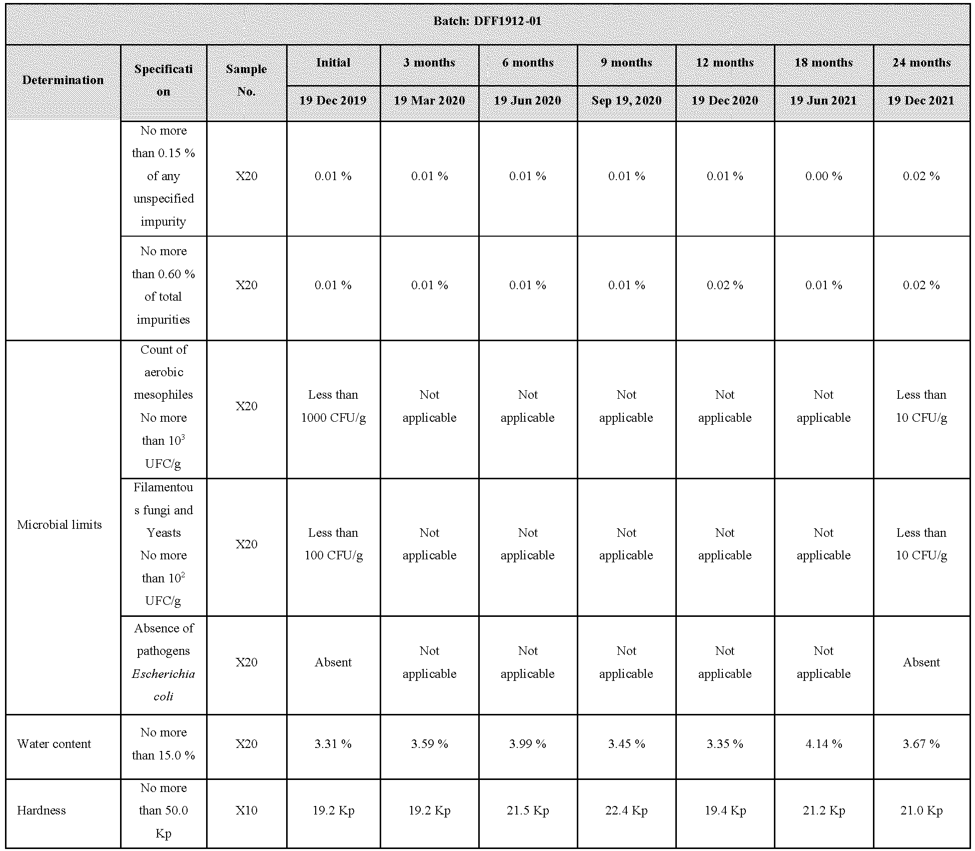

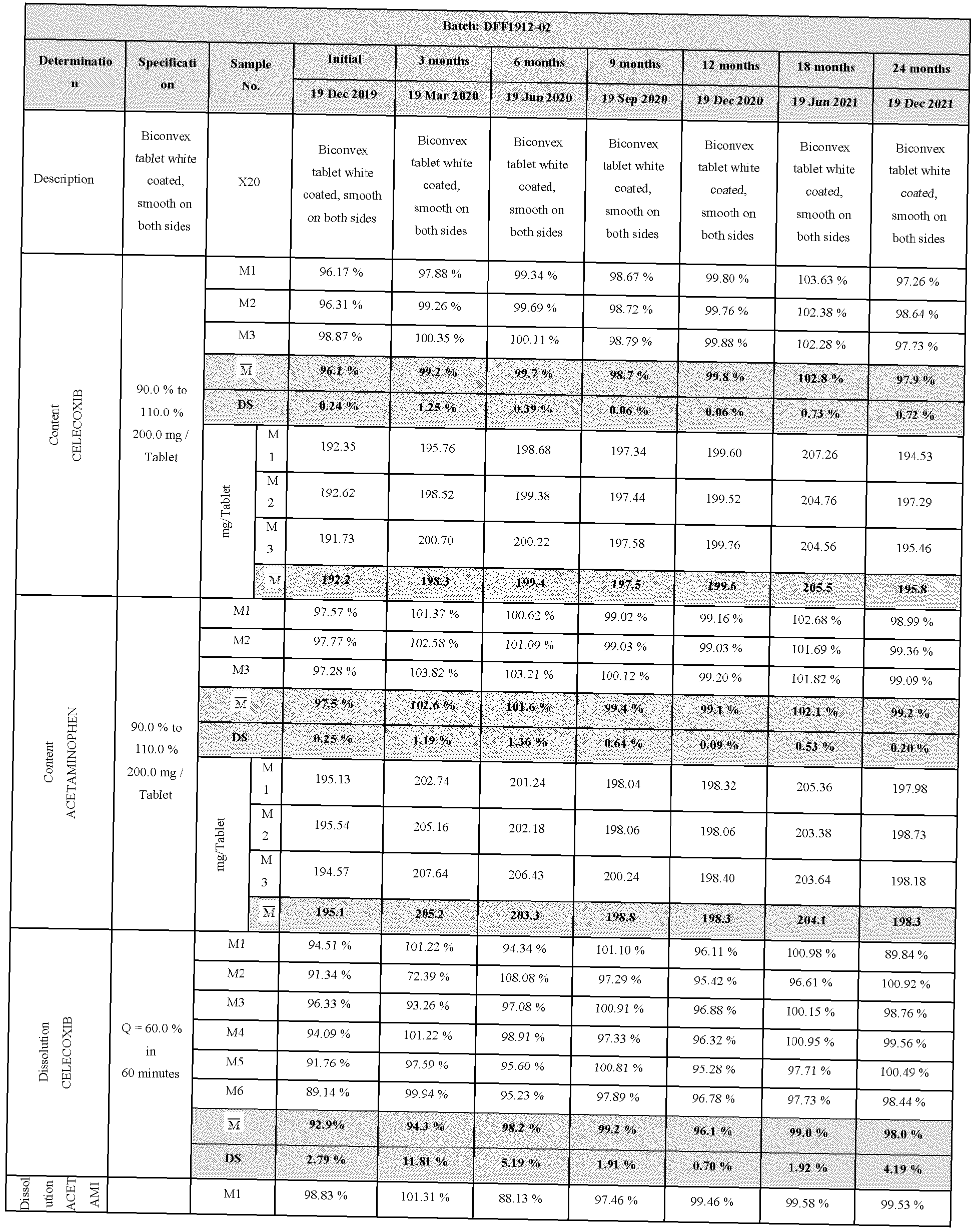

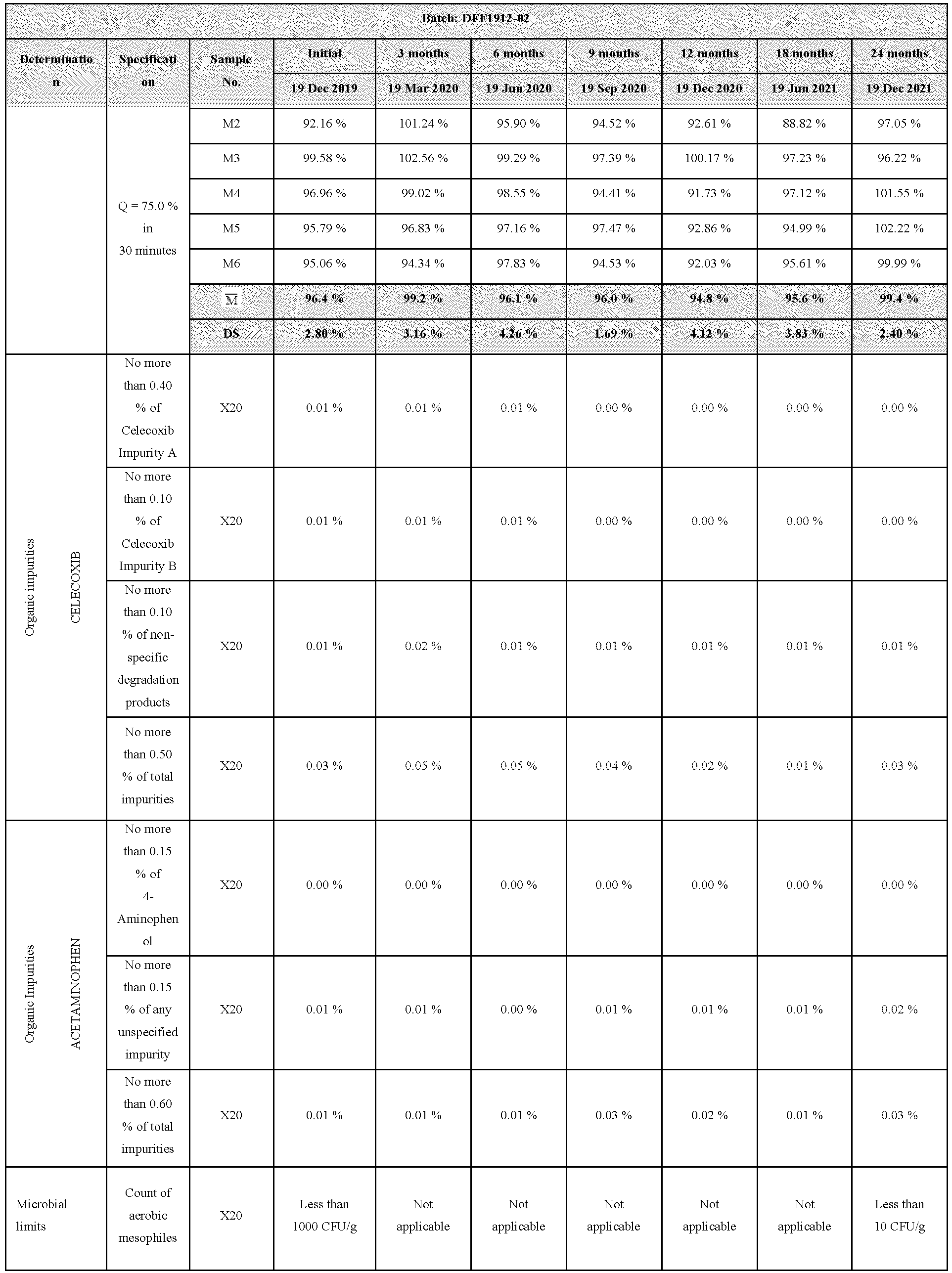

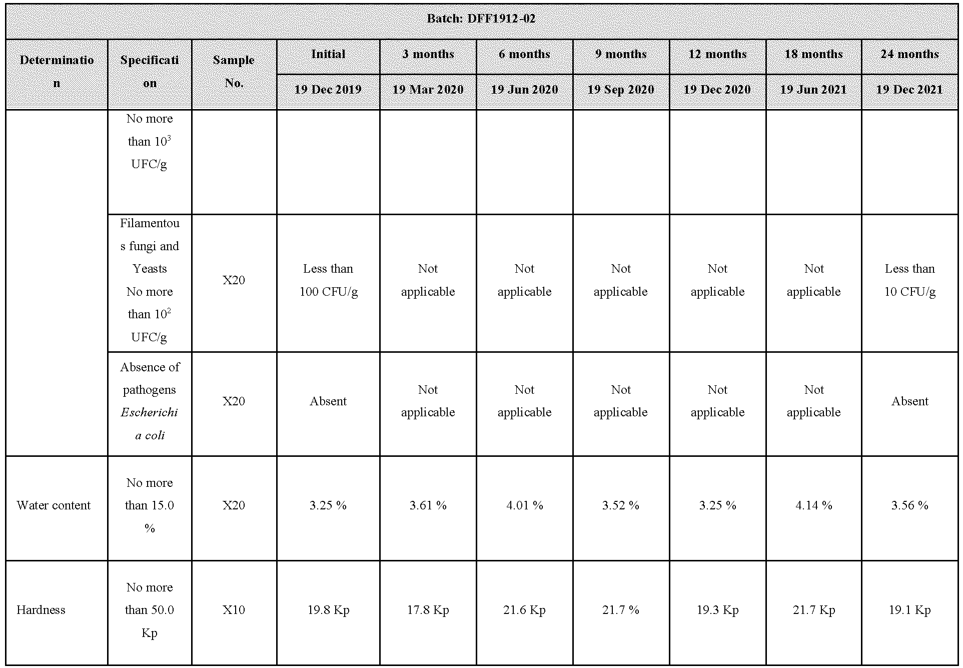

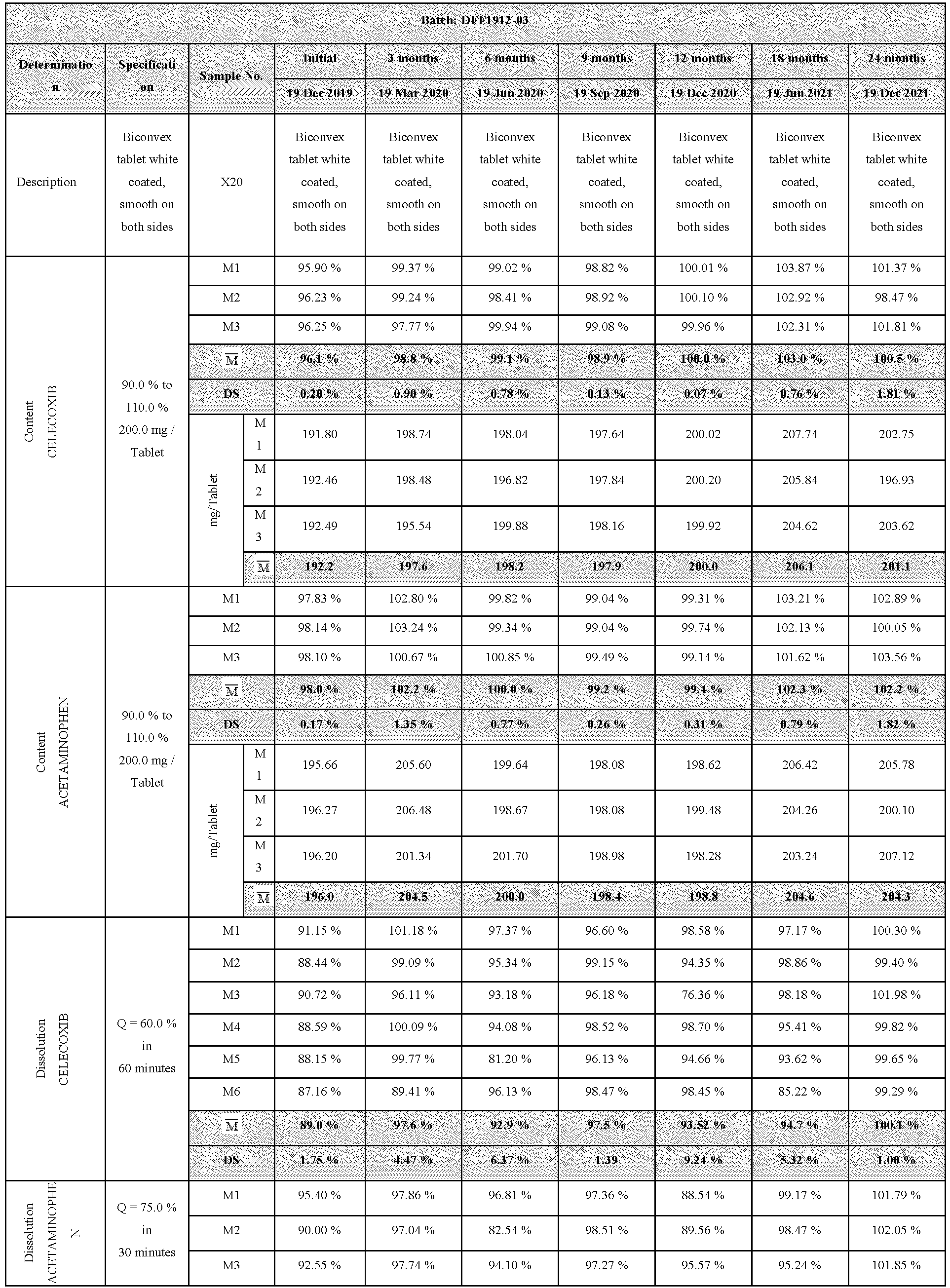

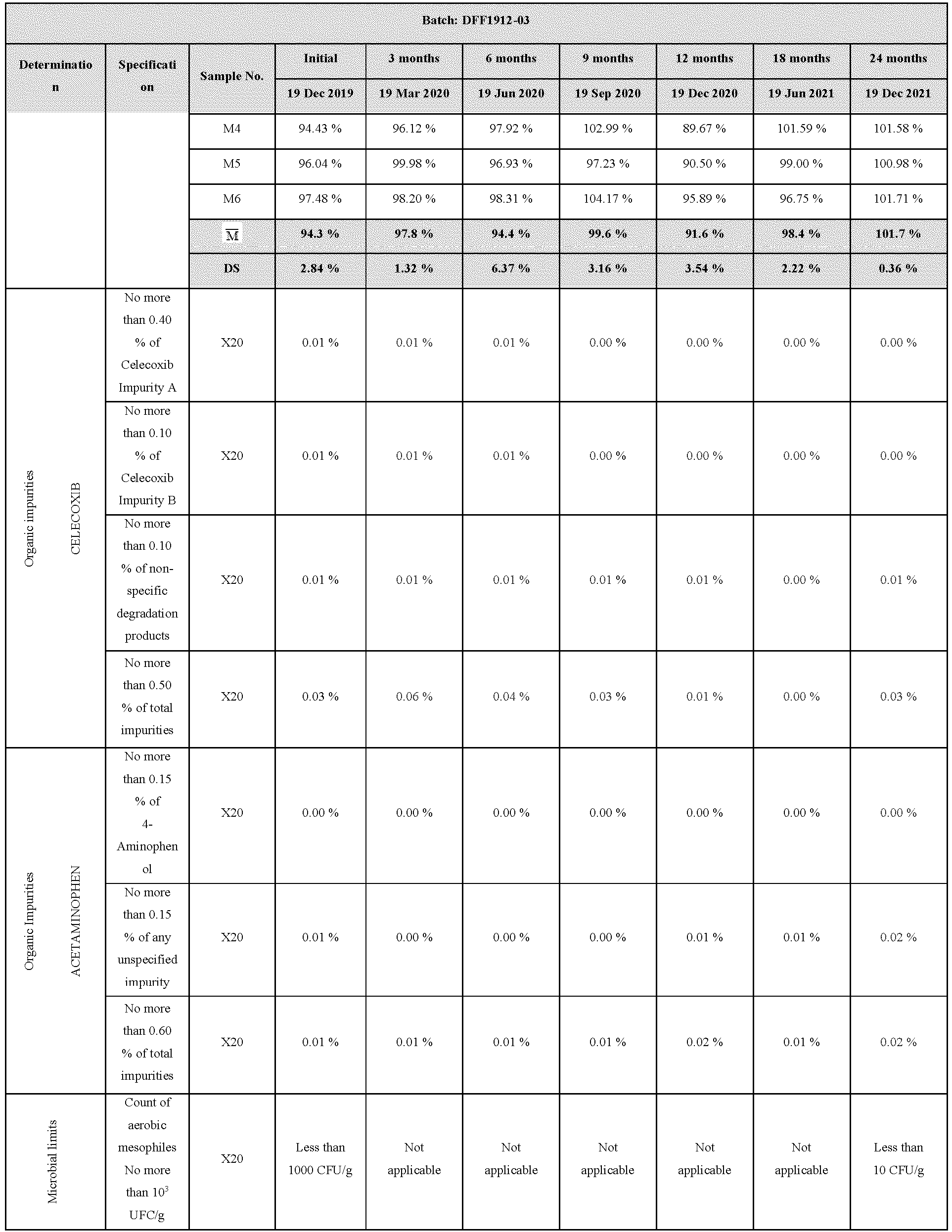

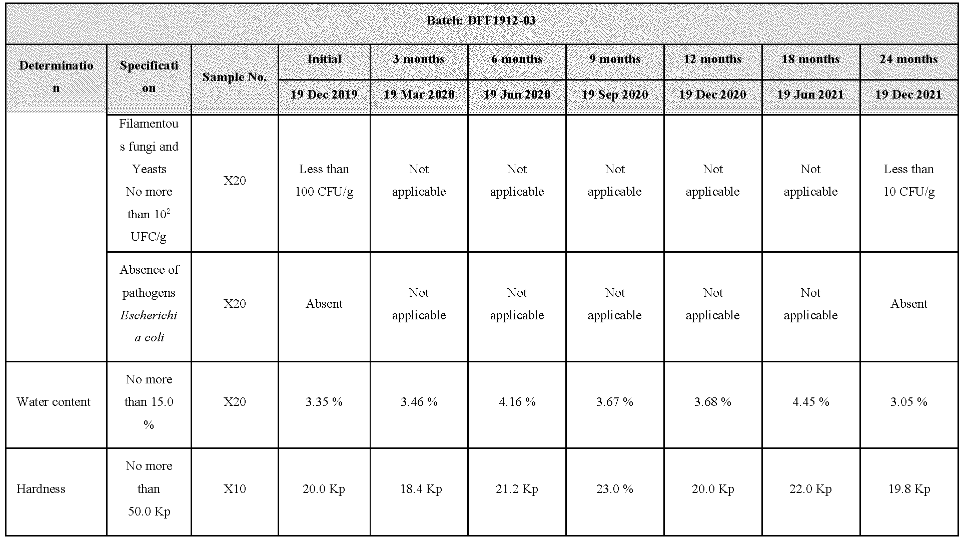

### Long-Term Stability Study (30 °C ± 2 °CZ 75% RH ± 5% RH) for Celecoxib/Acetaminophen [200 mg/500 mg]

The results obtained for batchs **DFF1911-13, DFF1911-14 and DFF1911-15** are presented at 24 months in long-term stability (30 °C ± 2 °C/75 % ± 5% RH).

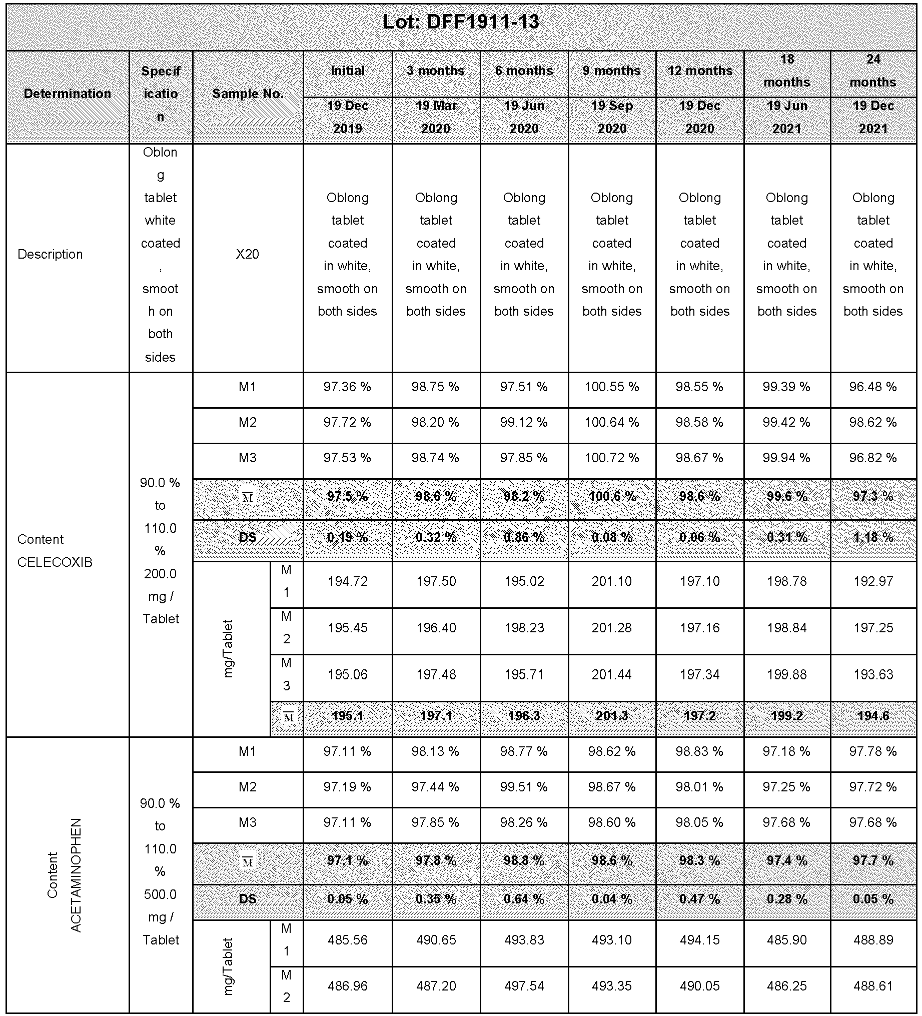

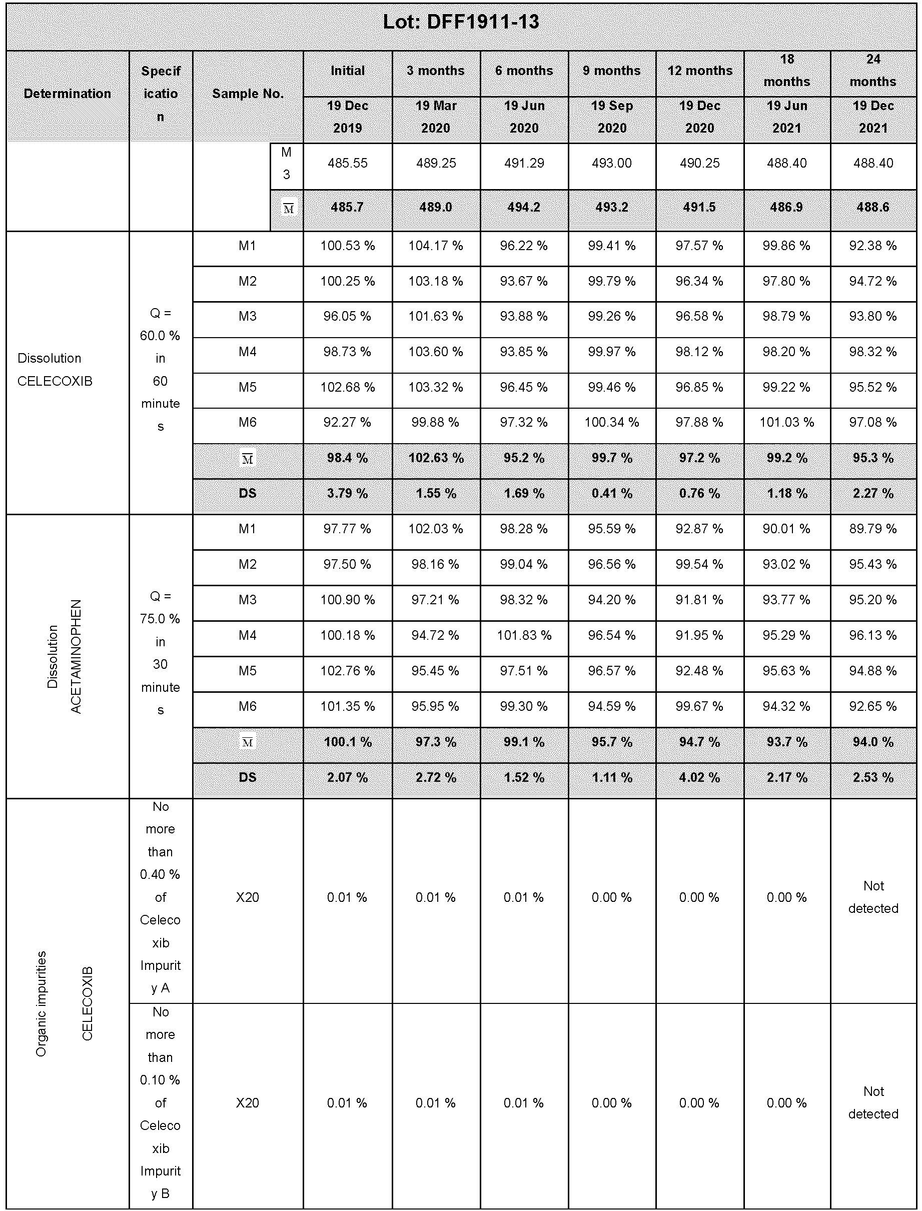

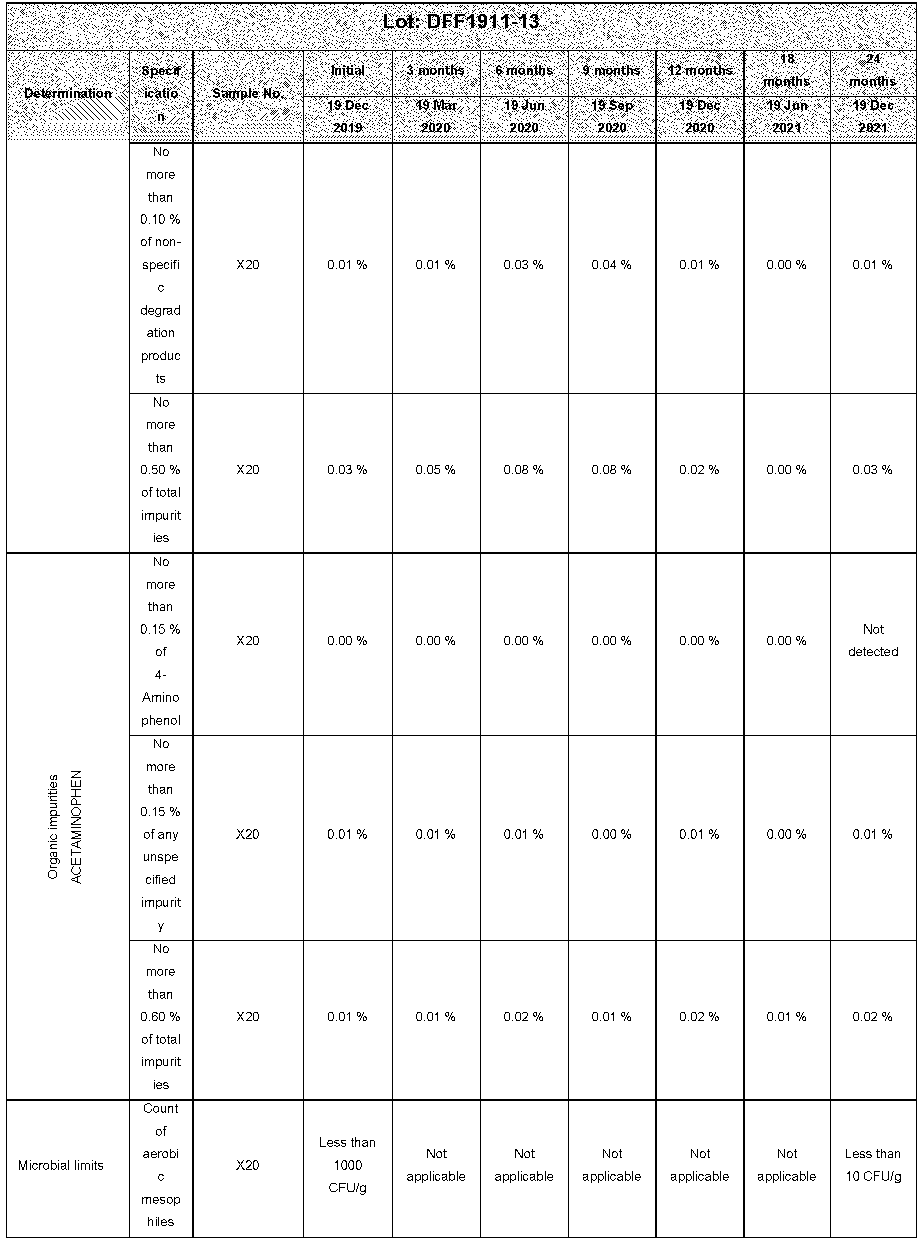

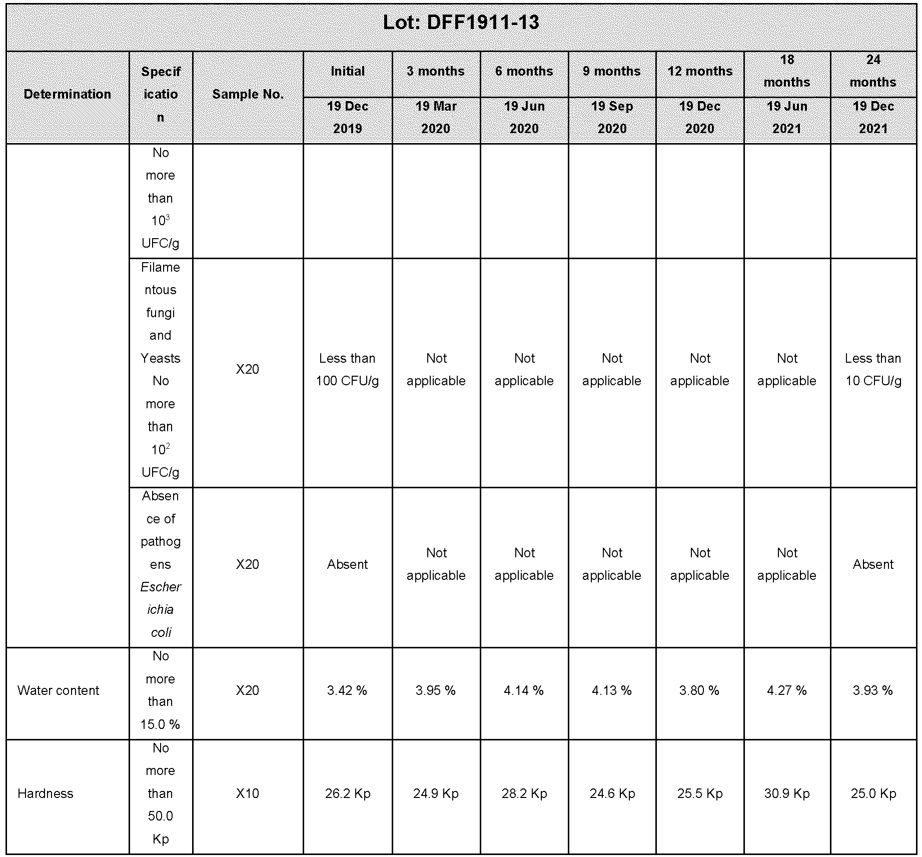

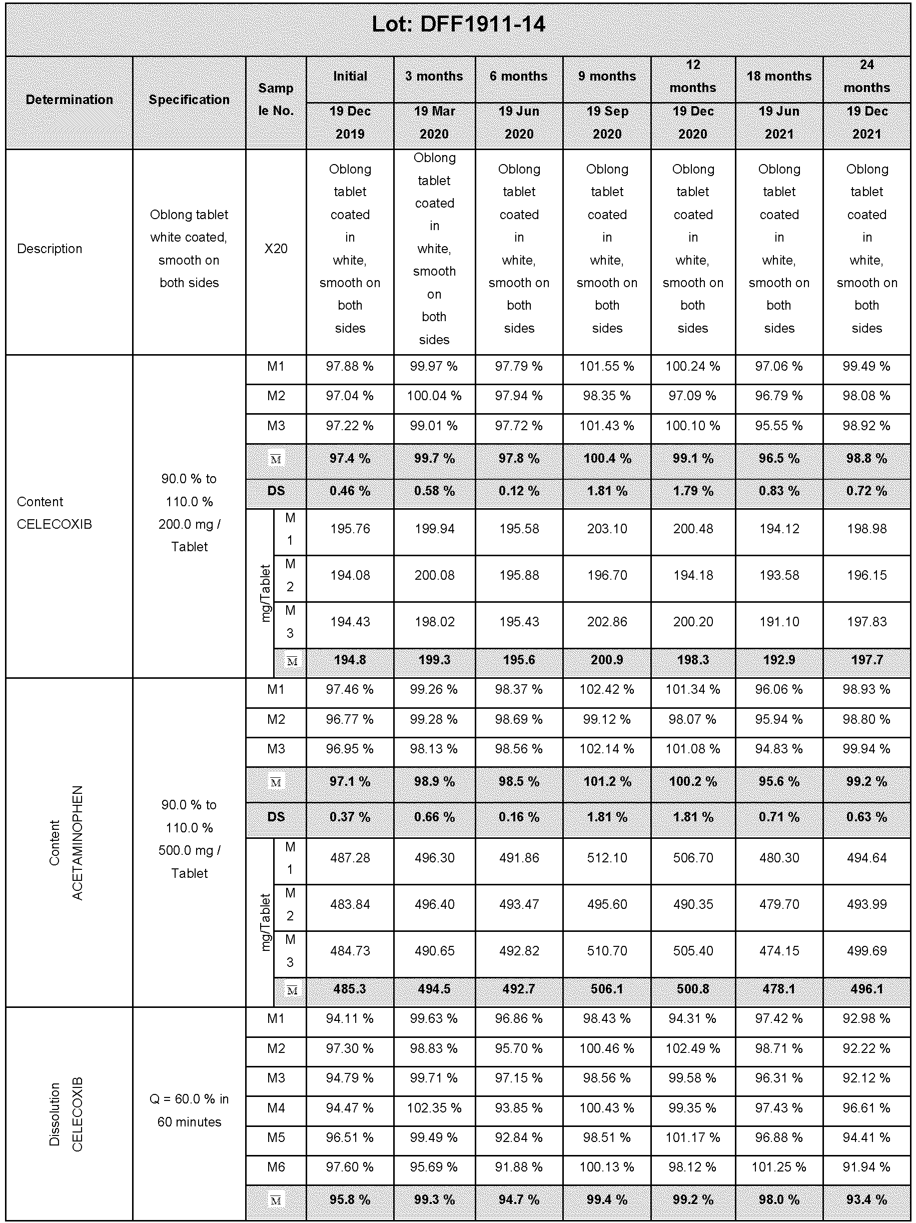

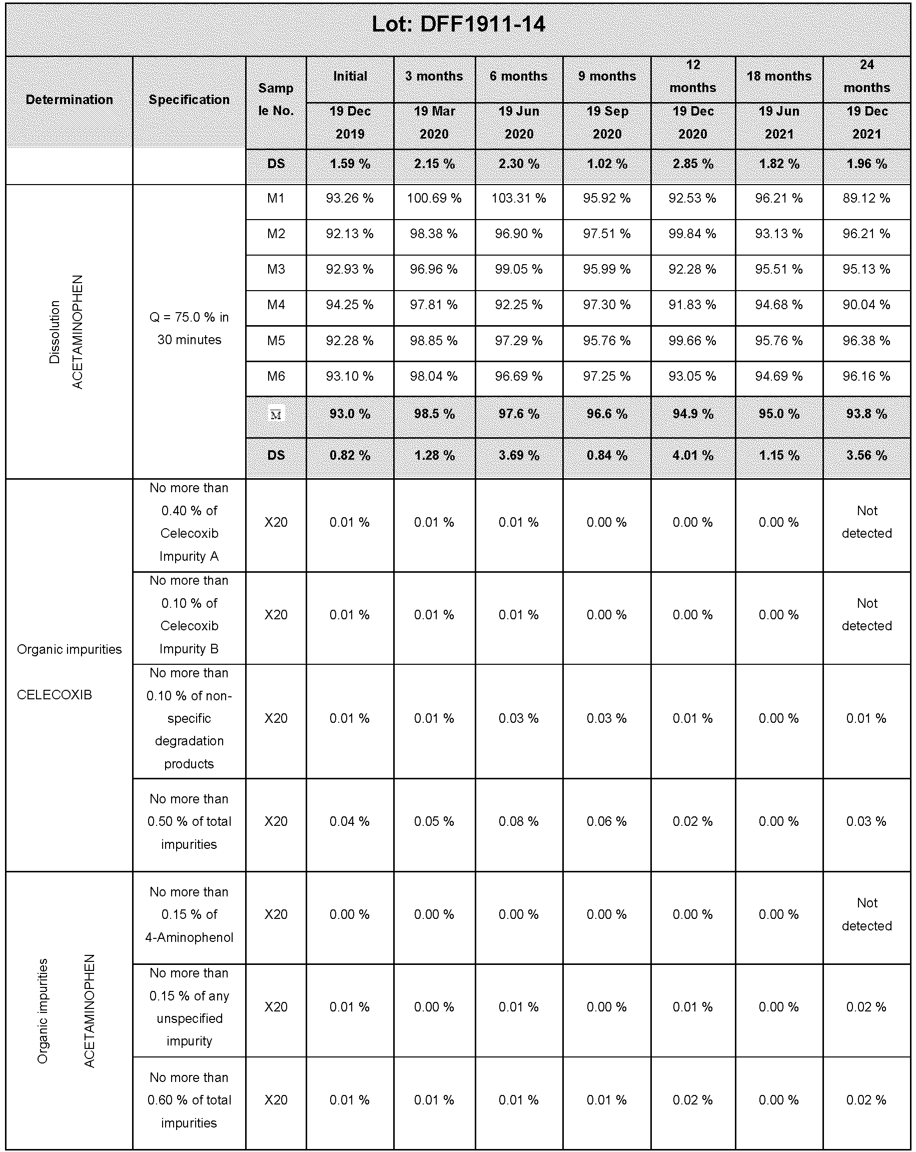

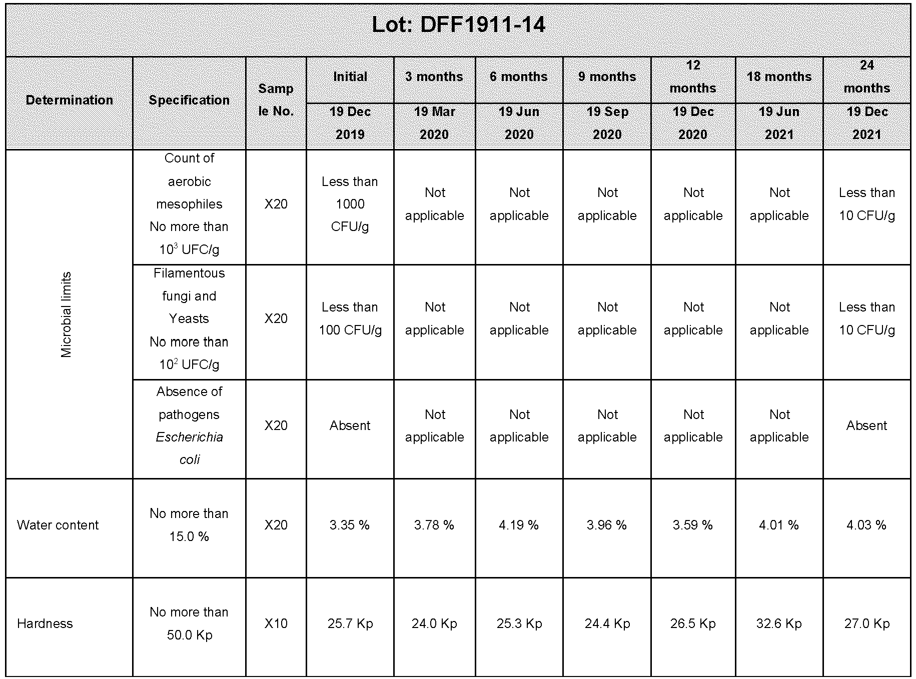

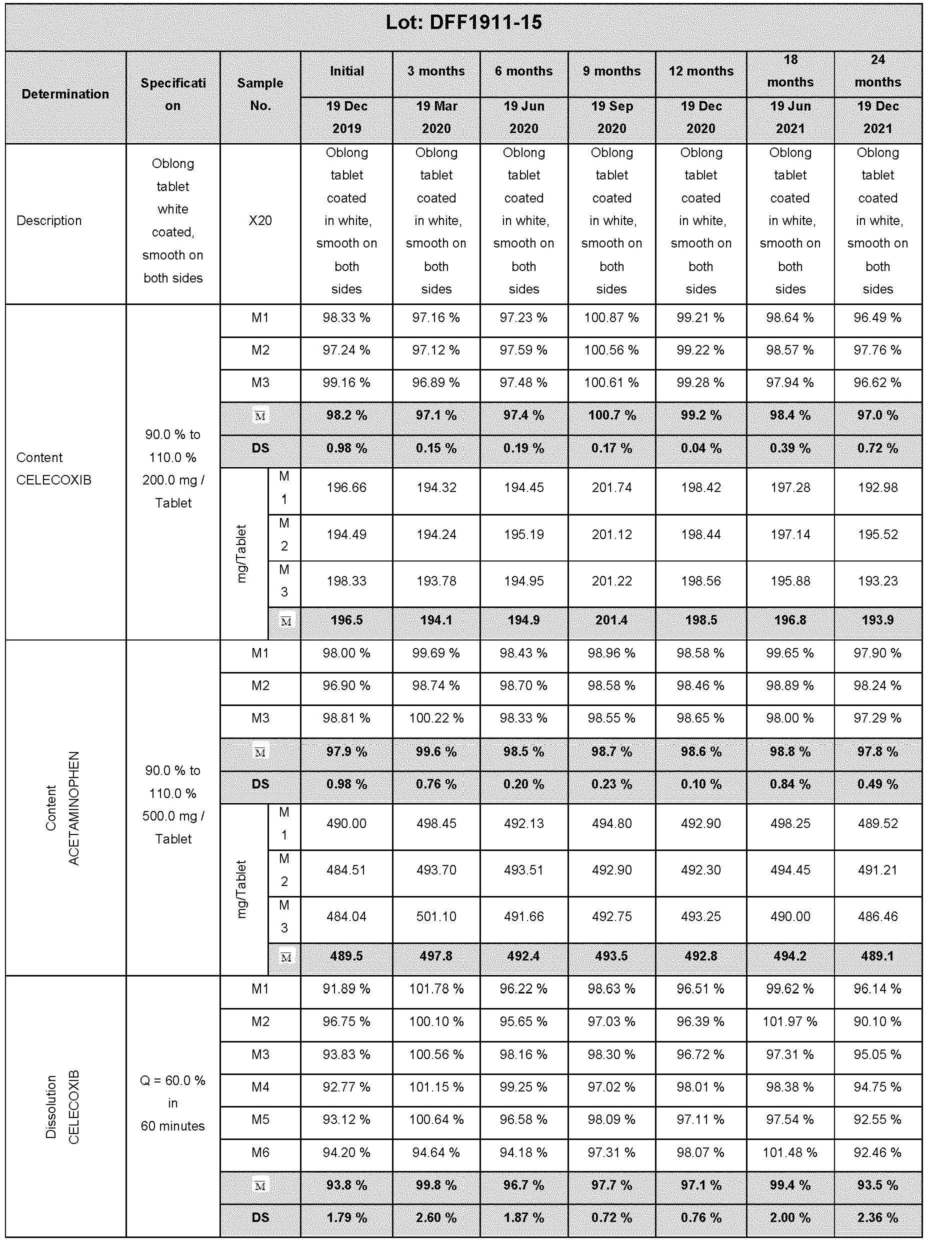

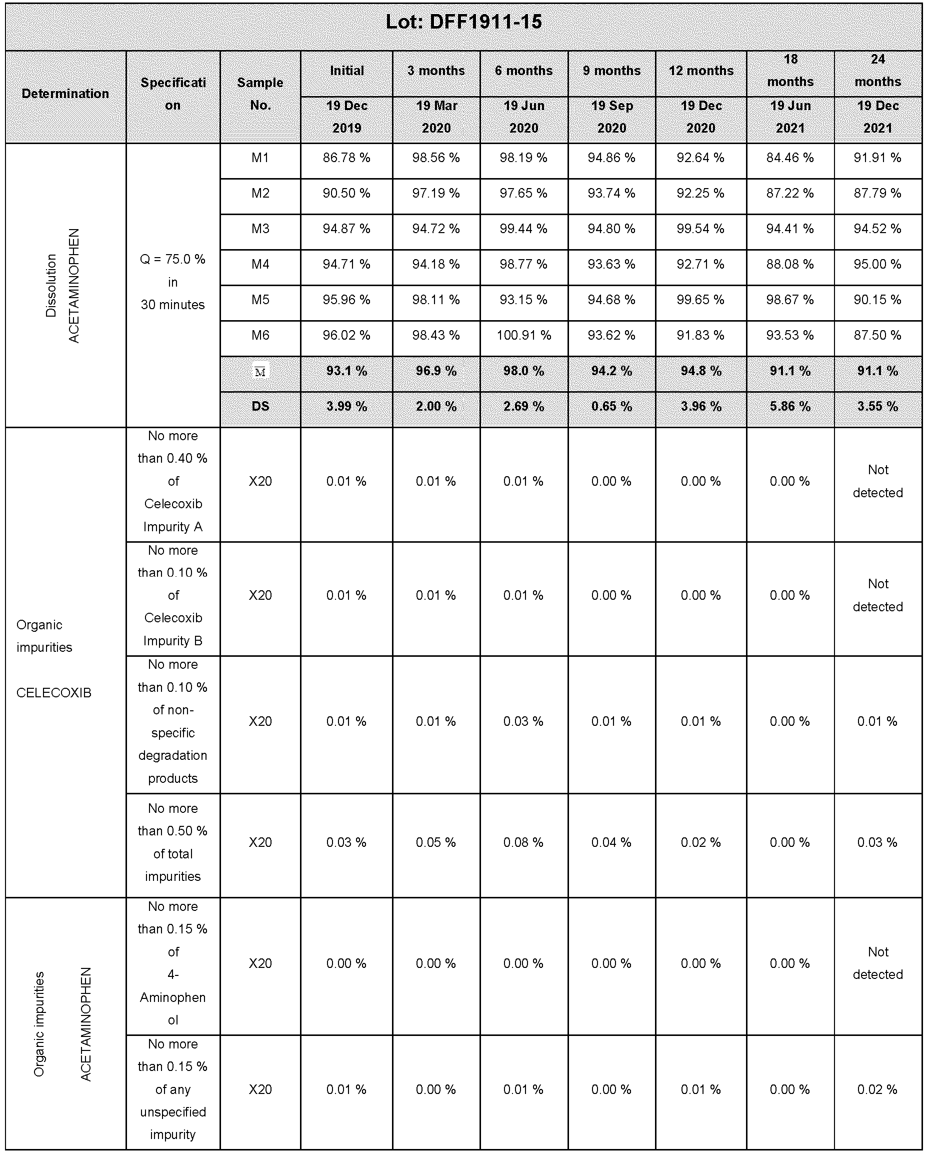

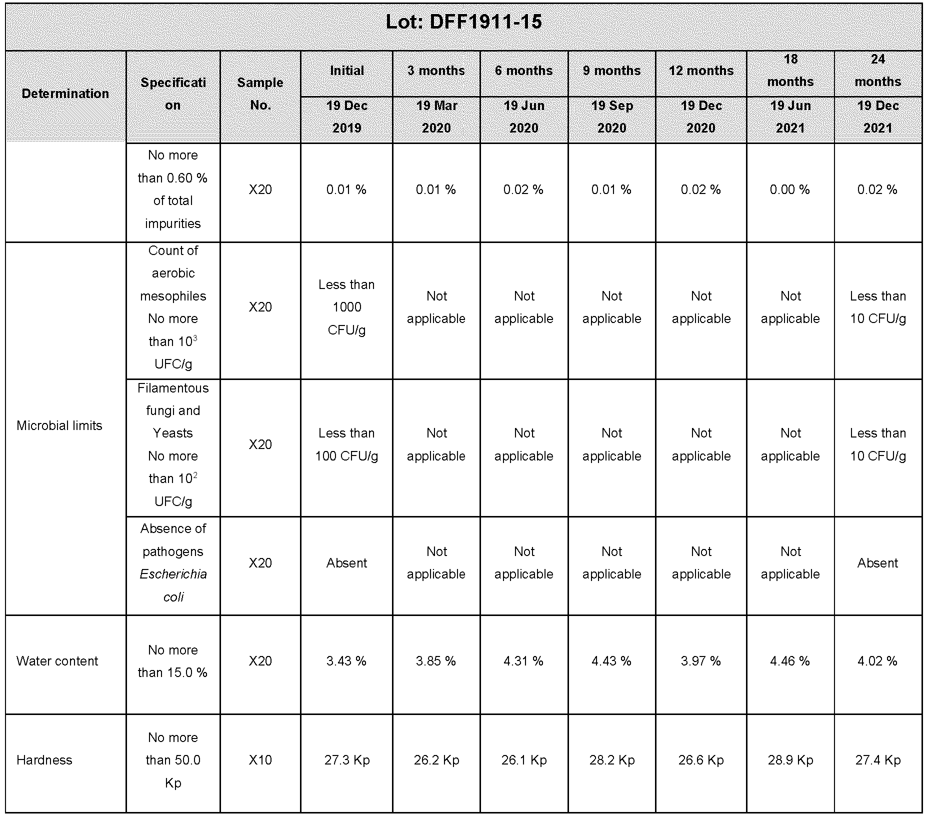

### Accelerated stability study (40 °C ± 2 °C/75 % RH ± 5% RH) for Celecoxib/Acetaminophen F200 mg/200 mg]

Batches **DFF1912-01, DFF1912-02 and DFF1912-03** of the product **Celecoxib** - **Acetaminophen 200 mg** / **200 mg Tablets,** subjected to the accelerated stability condition (40 °C) ± 2 °C / 75% RH ± 5% RH) for 6 months, demonstrated that they retain their chemical and physical characteristics within the established specifications, thus ensuring their Quality, Efficacy and Safety.

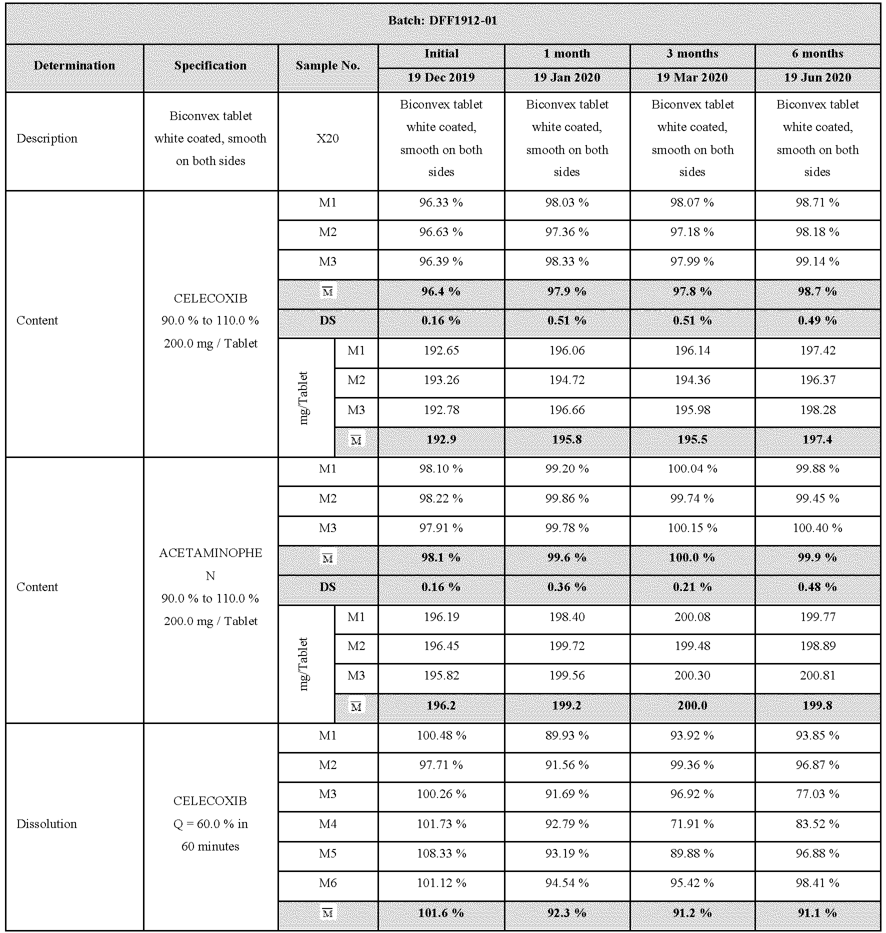

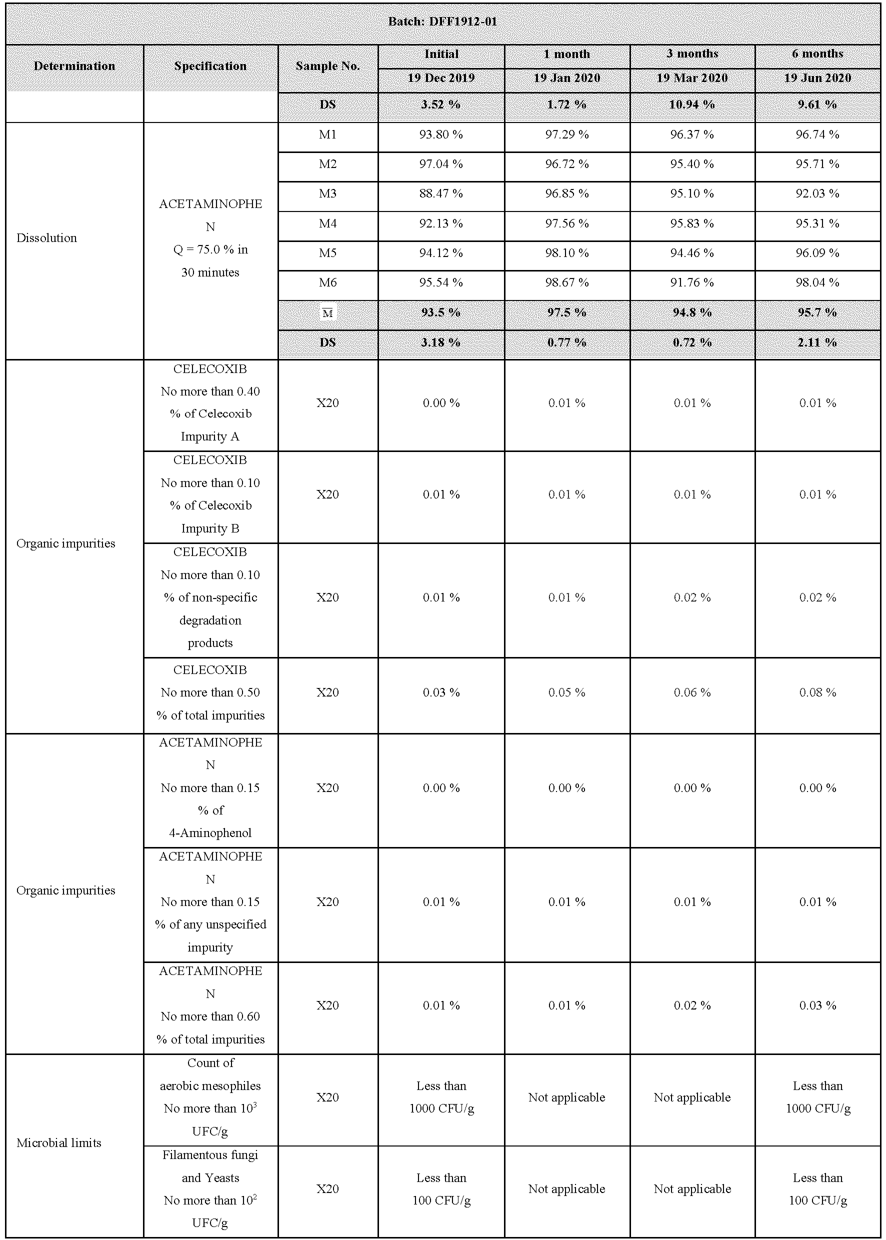

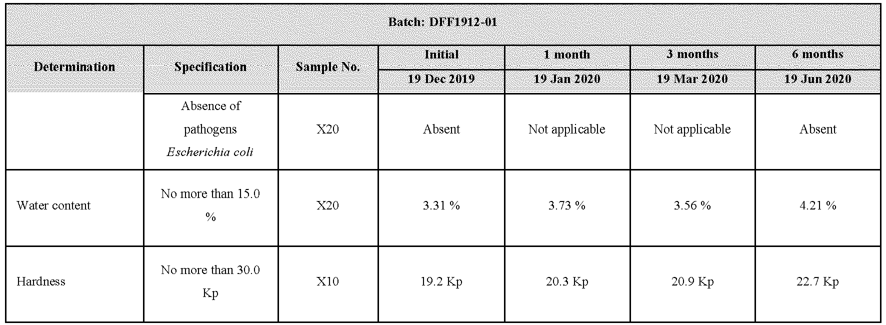

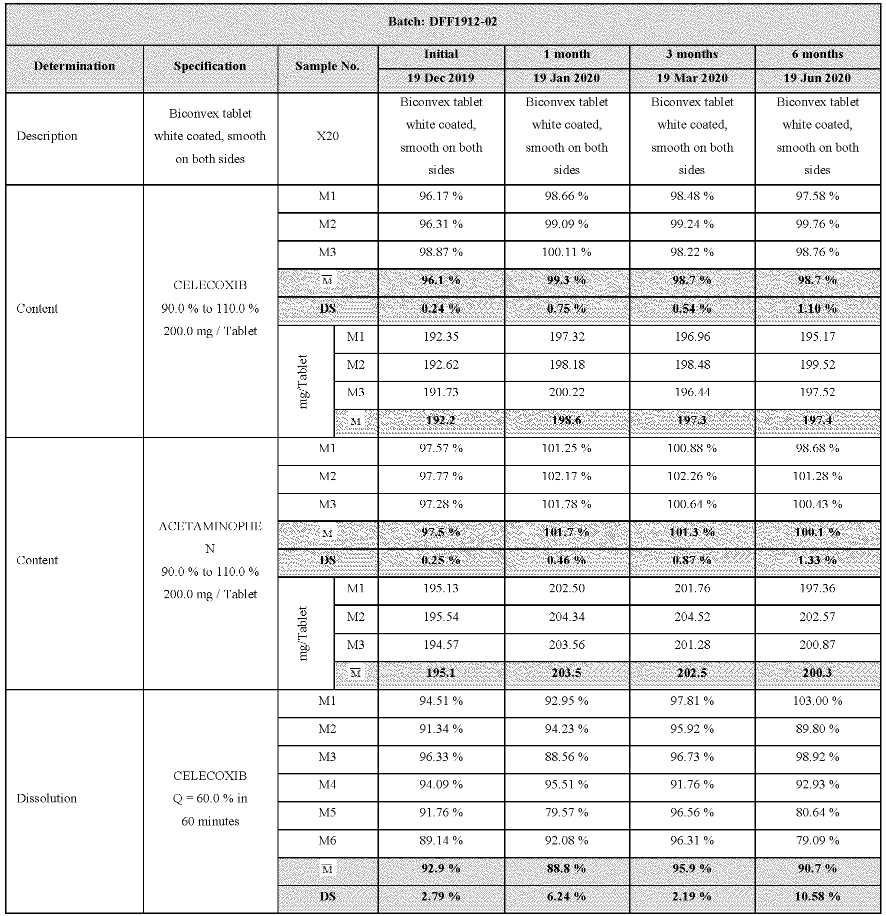

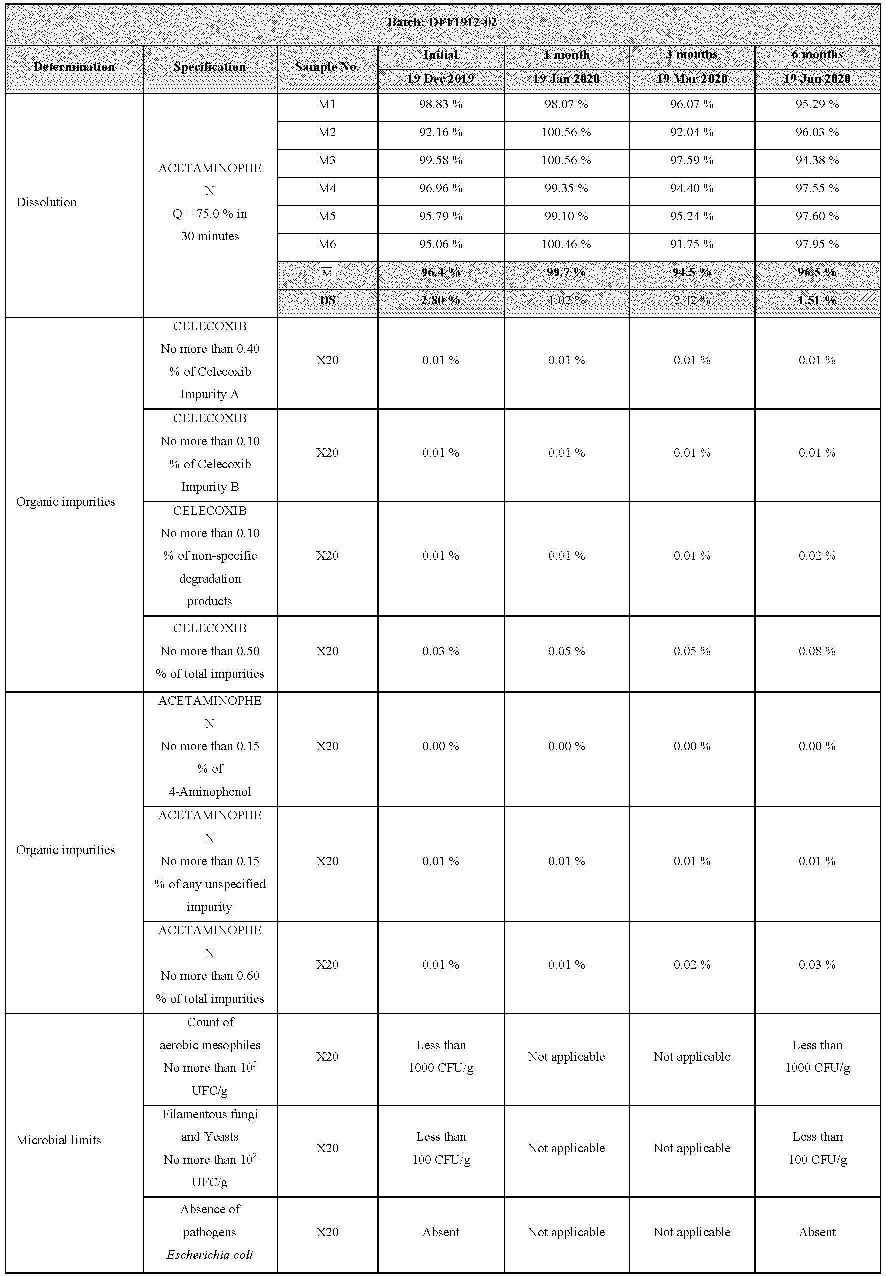

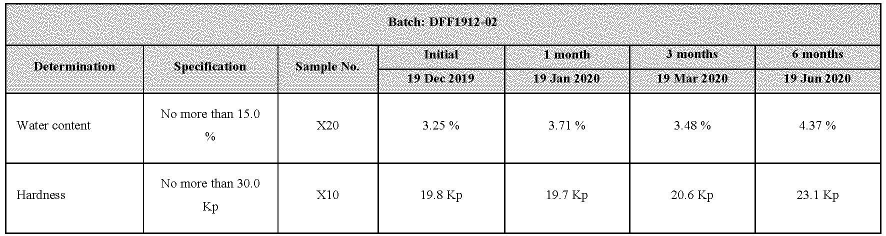

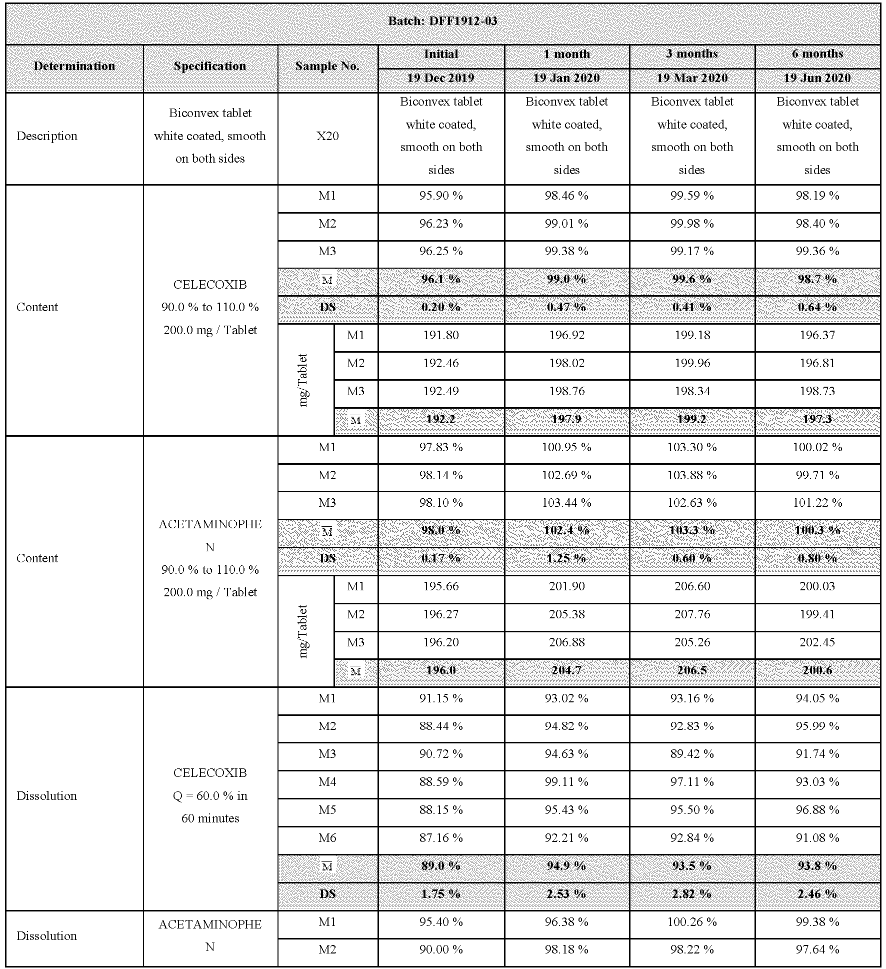

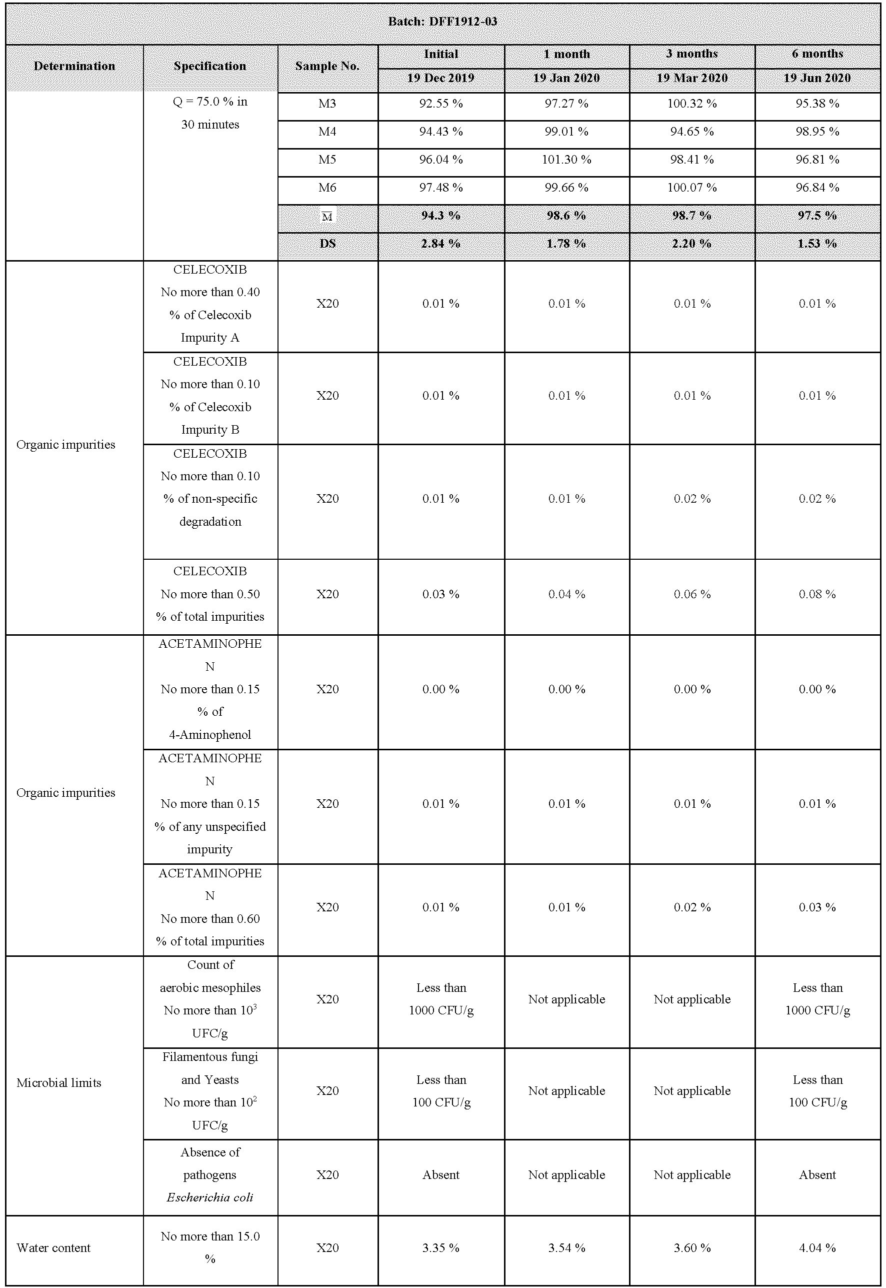

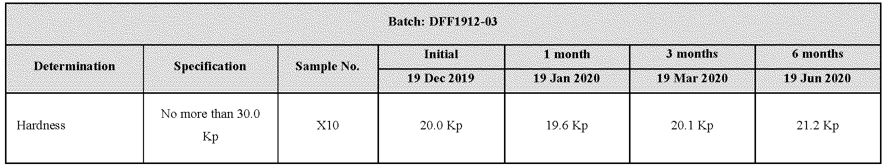

Accelerated stability study (40 °C ± 2 °C / 75% RH ± 5% RH) for Celecoxib/Acetaminophen F200 mg 1500 mg].

The results obtained for batches **DFF1911-13, DFF1911-14 and DFF1911-15 celecoxib** - **acetaminophen 200 mg / 500 mg Tablets** at 6 months at Accelerated stability (40 °C ± 2 °C / 75% RH ± 5% RH) are shown below.

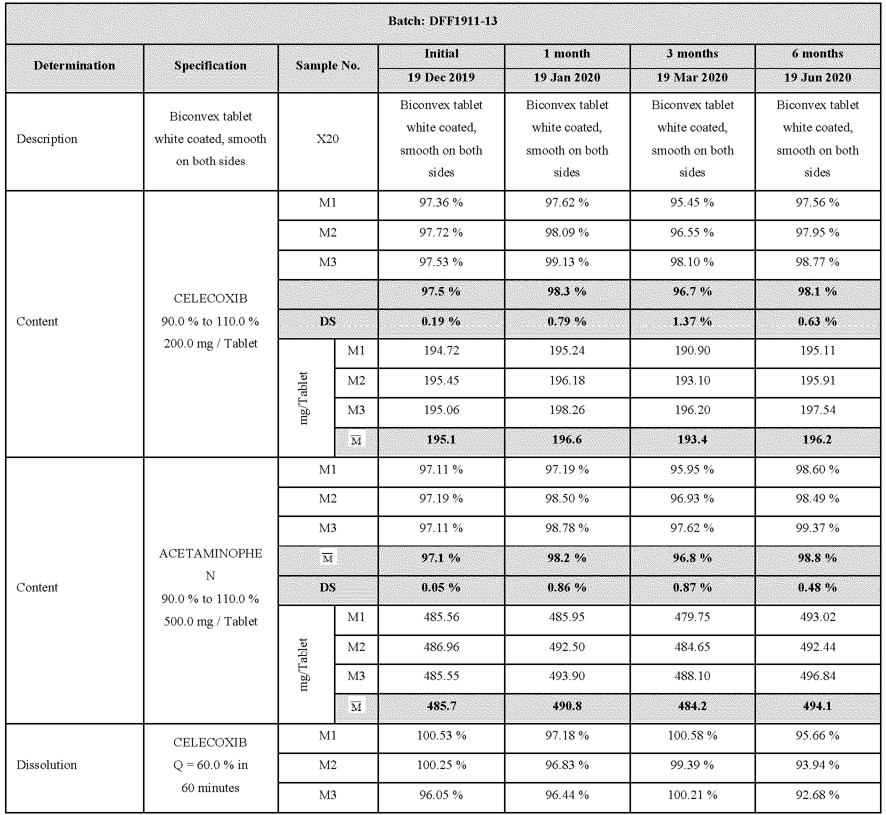

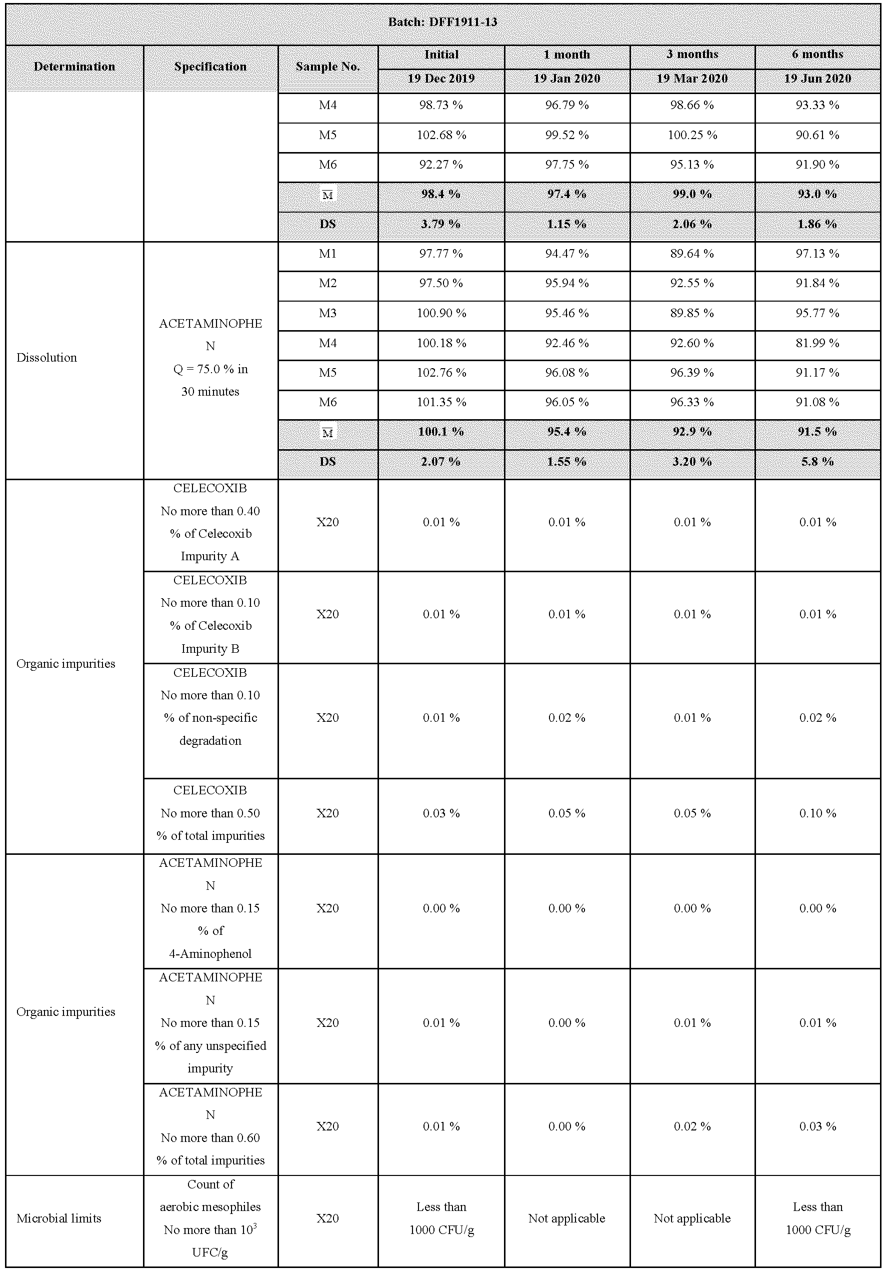

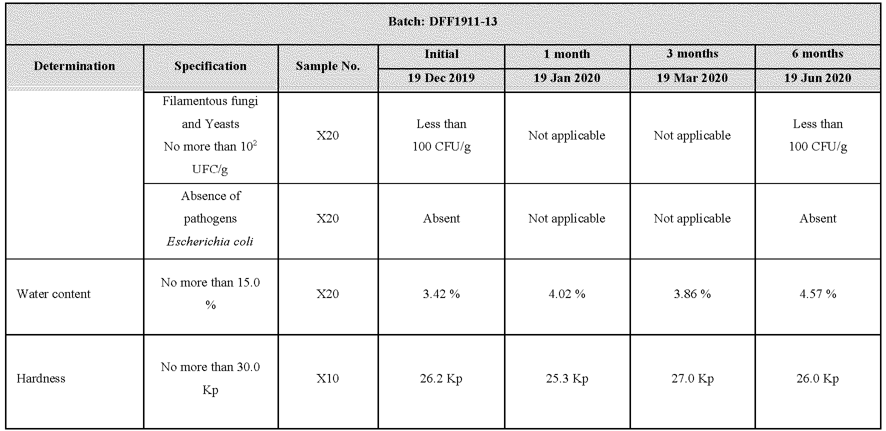

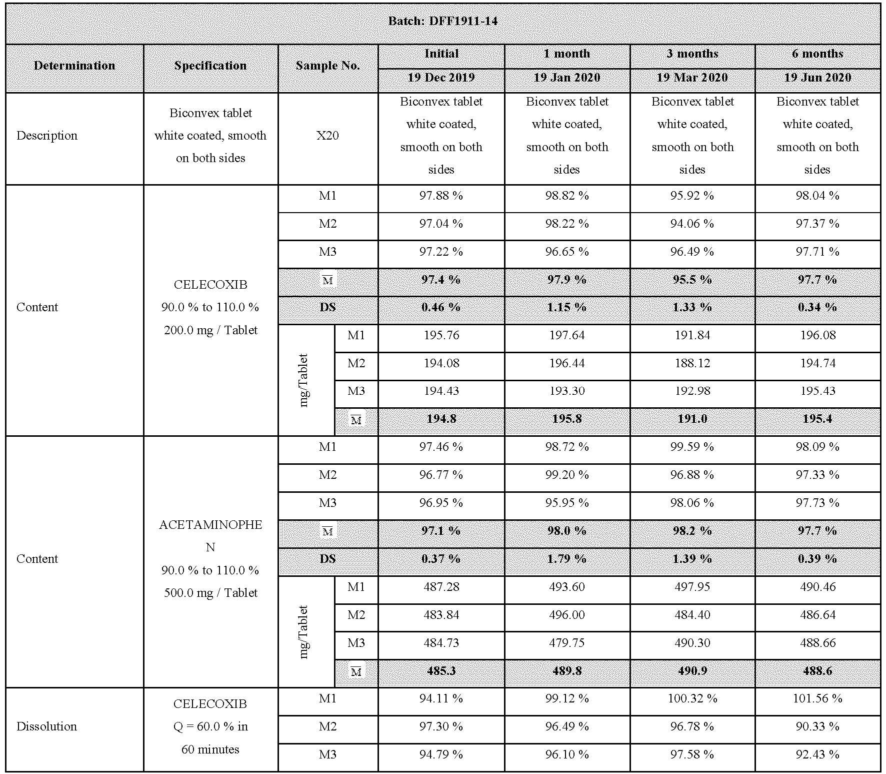

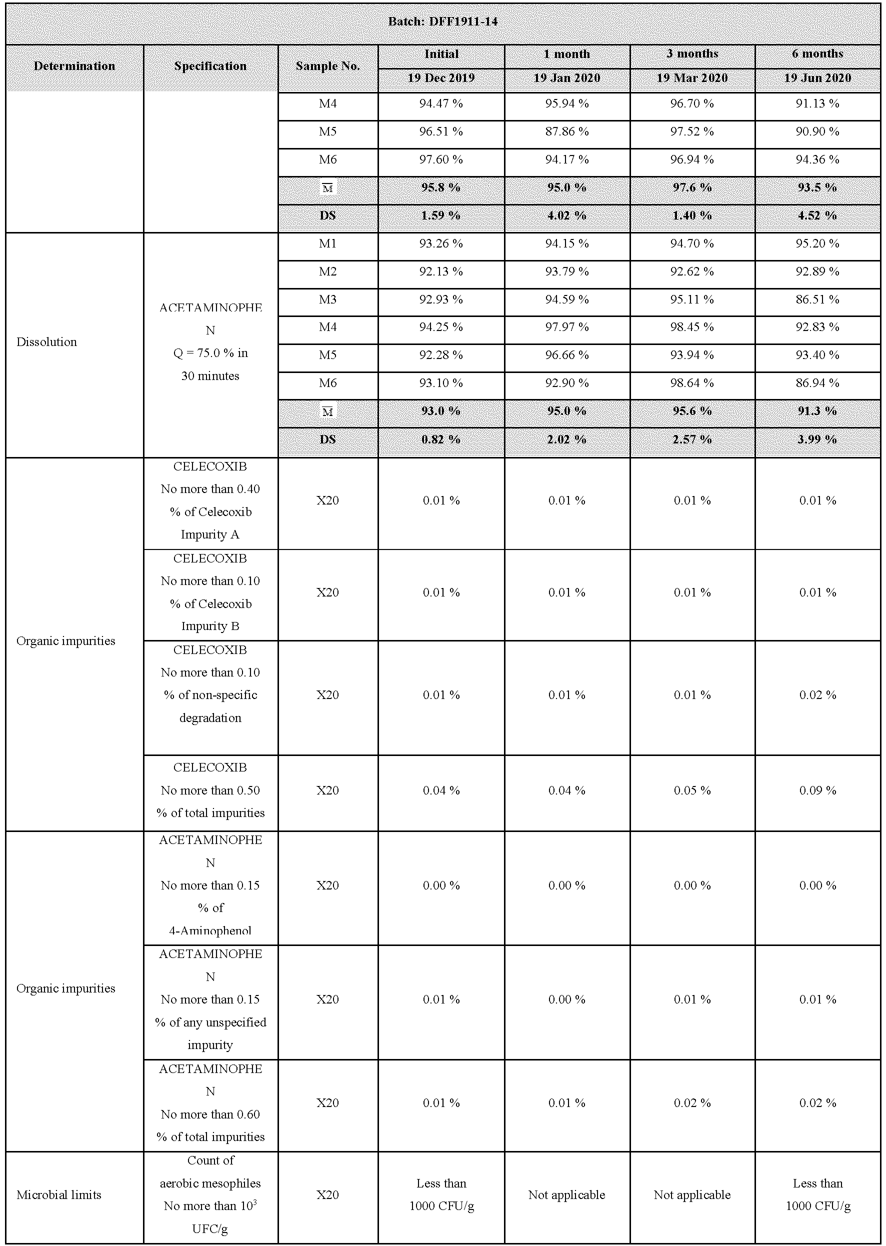

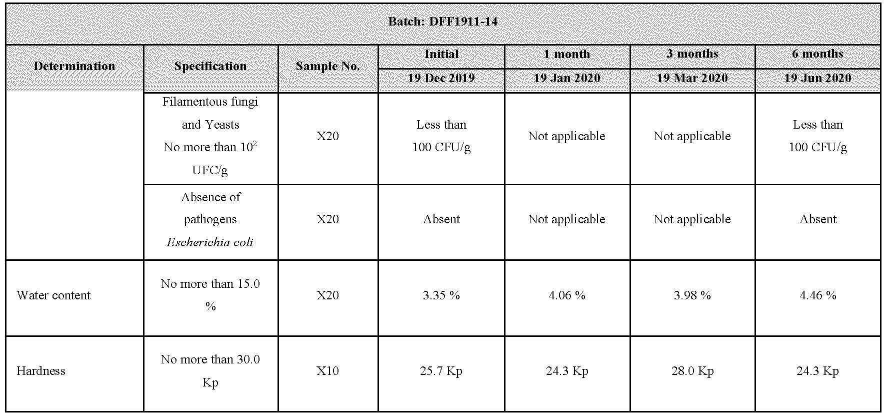

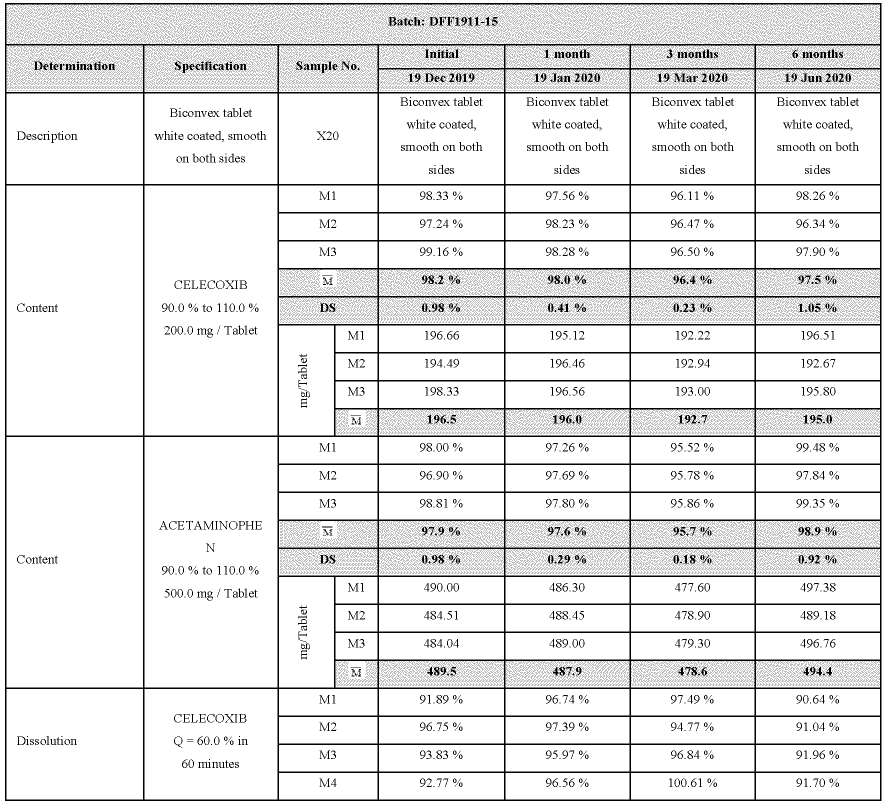

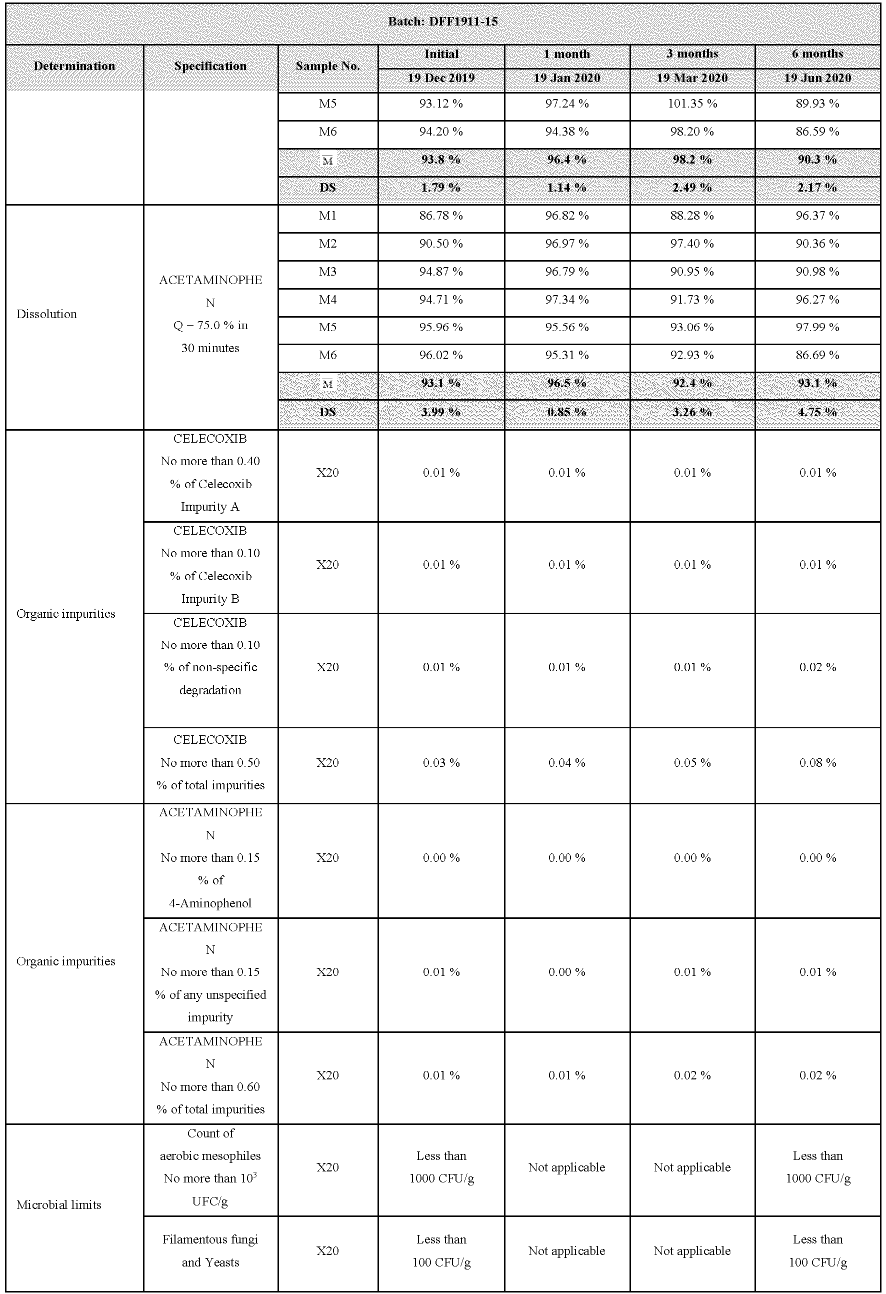

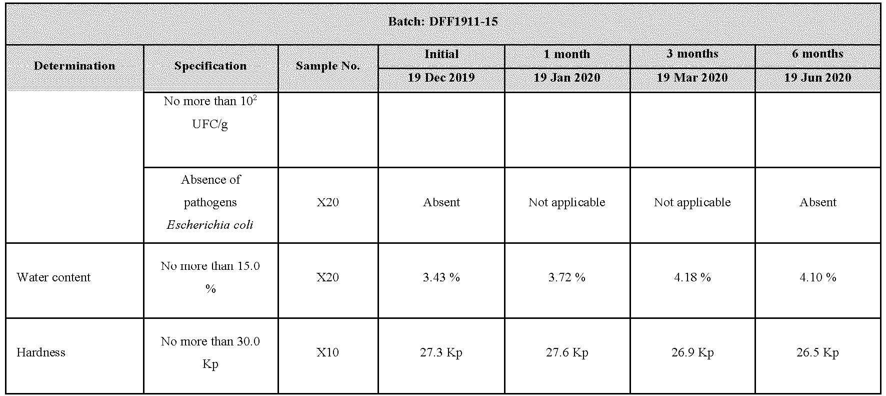

### Pharmacokinetic non-interaction study between Celecoxib 200 mg and Acetaminophen 500 mg administered individually or in combination, single dose in healthy subjects, both genders under fasting conditions.

In the present study, the pharmacokinetic profile (Cₘₐₓ and ABC₀₋ₜ) of the fixed-dose combination of celecoxib/acetaminophen 200 mg/500 mg, single dose, versus each component administered individually, in healthy subjects, of both sexes, under fasting conditions, to establish non-interaction of the drugs in combination.

**Test medicament and reference medicament.**

| | Test drug | Reference drug 1 | Reference drug 2 |
|---|---|---|---|
| International Nonproprietary Name | Celecoxib - Paracetamol | Celecoxib | Paracetamol |
| Generic Name | Celecoxib - Paracetamol | Celecoxib | Paracetamol |
| Distinctive Name | Celecoxib - Paracetamol | CELEBREX^{®} | TYLENOL^{®} |
| Pharmaceutical Form | Tablets | Pill | Tablets |
| Dose | 200 mg 1500 mg | 200 mg | 500 mg |
| Batch No. | 20B021G1 | AR3352 | AM5269 |
| Manufacturer | Laboratorios Silanes S.A. de C.V. | Pfizer, S.A. de C.V. | Janssen-Cilag, S.A. de C.V. |
| Expiration Date | February 2022 | October 2021 | May 2022 |

The study aims to establish the pharmacokinetic parameters of celecoxib and acetaminophen (paracetamol) calculated from the plasma levels of the research subjects after the administration of three medicaments: one in fixed combination at a single dose of celecoxib-paracetamol tablets 200 mg / 500 mg, respectively, versus the individual administration (monotherapy) of the reference drugs, celecoxib capsules 200 mg and paracetamol tablets 500 mg, under fasting conditions and to check if the 90 % confidence intervals for the test medicament and the reference medicaments are within the range 80 to 125 % for AUC₀₋ₜ and Cₘₐₓ, range cited in the protocol. In this way, equivalence in bioavailability is established.

The study was longitudinal, prospective, open, crossover, 3x6x3, three treatments, three periods, six sequences, single dose, of celecoxib / acetaminophen 200 mg 1500 mg administered orally in combination or individually according to randomization, under fasting conditions, in a healthy Mexican male and female population with a washout time for drug elimination of one week (07 days) between each period.

The study was conducted in two groups and two periods. The study began and ended with 42 male (14) and female (28) research subjects. After fasting for at least 10 hours, a blood sample was taken at time 0.00 hours, and a single dose of celecoxib acetaminophen tablets 200 mg/500 mg was administered as a single dose of the test medicament or the reference drug as monotherapy (Celebrex^{®} celecoxib 200 mg or Tylenol^{®} paracetamol 500 mg) according to prior randomization orally with 250 mL of water at room temperature. After administration of the corresponding medicament, another 20 samples were taken at the following times: 0.16, 0.33, 0.50, 0.75, 1.00, 1.25, 1.50, 1.75, 2.00, 2.50, 3.00, 3.50, 4.00, 6.00, 8.00, 10.00, 12.00, 24.00, 36.00- and 48.00-hours post-dose.

Analytical methods for plasma quantification of celecoxib and acetaminophen were developed with bibliographic support from the publications of Akhtar M. *et al.,* Emami J. *et. al.,* Paulson S. et. al. and Shep D., *et. al.* Analysis of celecoxib in plasma samples for the celecoxib - acetaminophen study was performed by protein precipitation with subsequent HPLC/MS-MS analysis over a concentration range of 20,000 to 2000,000 ng/mL. Acetaminophen analysis was performed by protein precipitation, with subsequent HPLC/MS analysis in a concentration range of 0.250 to 20,000 µg/mL.

The average values of the pharmacokinetic parameters, their respective arithmetic means, standard deviations, coefficient of variation, geometric mean, minimum, median, and maximum of the estimated pharmacokinetic parameters of the reference medicament (R) and the test medicament (P) sampling were as follows:

### Celecoxib

Descriptive statistics of celecoxib pharmacokinetic parameters in research subjects.

| Pharmacokinetic parameter | Arithmetic mean | | Standard deviation | | Variation coefficient | | Geometric mean | | Minimum | | Mean | | Maximum | |
|---|---|---|---|---|---|---|---|---|---|---|---|---|---|---|
| | T2 | T1 | T2 | T1 | T2 | T1 | T2 | T1 | T2 | T1 | T2 | T1 | T2 | T1 |
| ABC₀₋ₜ (µg/mL*h) | 5593.760 | 5716.008 | 1617.086 | 1788.515 | 0.289 | 0.312 | 5362.640 | 5432.471 | 2761.864 | 2099.279 | 5193.131 | 5492.161 | 9035.787 | 10251.581 |
| ABC_{0-∞} (µg/mL*h) | 6472.077 | 6570.640 | 2231.034 | 2158.606 | 0.344 | 0.328 | 6171.724 | 6253.942 | 3168.173 | 2680.619 | 5855.489 | 6185.715 | 15982.778 | 1476.423 |
| Cmax (µg/mL) | 602.685 | 636.822 | 301.486 | 274.954 | 0.500 | 0.431 | 534.889 | 582.070 | 190.079 | 165.480 | 597.100 | 583.979 | 1528.271 | 1518.389 |
| Vd/F (mL) | 641.163 | 615.701 | 302.422 | 276.955 | 0.471 | 0.449 | 587.246 | 572.706 | 259.987 | 286.720 | 565.970 | 528.961 | 1691.598 | 1860.688 |
| Cl/F (mL/h) | 33.827 | 33.629 | 9.958 | 11.338 | 0.294 | 0.337 | 32.405 | 31.979 | 12.513 | 14.107 | 34.157 | 32.349 | 63.127 | 74.609 |
| Kₑ (1/h) | 0.059 | 0.060 | 0.021 | 0.022 | 0.355 | 0.373 | 0.055 | 0.055 | 0.015 | 0.021 | 0.060 | 0.060 | 0.097 | 0.120 |
| T_{1/2} (h) | 13.965 | 13.501 | 7.930 | 6.108 | 0.567 | 0.452 | 12.560 | 12.413 | 7.110 | 5.762 | 11.529 | 11.376 | 44.289 | 32.530 |
| Tmax (h) | 2.476 | 2.505 | 1.101 | 1.602 | 0.4444 | 0.639 | 2.251 | 2.070 | 1.000 | 0.750 | 2.000 | 1.875 | 6.000 | 8.000 |

### Acetaminophen

Descriptive statistics of acetaminophen pharmacokinetic parameters in research subjects.

| Pharmacokinetic parameter | Arithmetic mean | | Standard deviation | | Variation coefficient | | Geometric mean | | Minimum | | Mean | | Maximum | |
|---|---|---|---|---|---|---|---|---|---|---|---|---|---|---|
| | T3 | T1 | T3 | T1 | T3 | T1 | T3 | T1 | T3 | T1 | T3 | T1 | T3 | T1 |
| ABC₀₋ₜ (µg/mL*h) | 33.483 | 33.369 | 11.981 | 10.409 | 0.357 | 0.311 | 31.613 | 31.832 | 16.694 | 15..197 | 31.735 | 31.766 | 69.210 | 65.520 |
| ABC_{0-∞} (µg/mL*h) | 35.535 | 35.489 | 12.539 | 10.823 | 0.352 | 0.304 | 33.563 | 33.897 | 17.874 | 16.175 | 34.288 | 34.568 | 71.505 | 67.029 |
| Cmax ( µg/mL) | 9.607 | 10.532 | 3.119 | 3.534 | 0.324 | 0.335 | 9.081 | 9.938 | 4.038 | 4.404 | 9.350 | 9.769 | 15.822 | 17.075 |
| Vd/F (mL) | 70474.688 | 73926.297 | 22695.394 | 47854.810 | 0.322 | 0.647 | 67532.742 | 66963.316 | 35360.898 | 39563.081 | 66345.811 | 63928804 | 167768.971 | 347608.187 |
| Cl/F (mL/h) | 15736.801 | 15480.178 | 5222.168 | 5081.167 | 0.331 | 0.328 | 14897.040 | 14750.421 | 6992.431 | 7459.446 | 14582.149 | 14466.461 | 27972.660 | 30910.131 |
| Kₑ (1/h) | 0.232 | 0.232 | 0.065 | 0.059 | 0.280 | 0.256 | 0.220 | 0.220 | 0.062 | 0.042 | 0.244 | 0.238 | 0.350 | 0.352 |
| T_{1/2} (h) | 3.407 | 3.483 | 1.737 | 2.373 | 0.509 | 0.681 | 3.142 | 3.146 | 1.977 | 1.967 | 2.835 | 2.908 | 11.068 | 16.445 |
| Tmax (h) | 1.260 | 0.815 | 0.969 | 0.429 | 0.768 | 0.527 | 0.991 | 0.715 | 0.330 | 0.330 | 1.000 | 0.750 | 4000 | 2.000 |

### 90% confidence interval

The 90% confidence intervals for the logarithmic transformation of Cₘₐₓ, ABC₀₋ₜ, ABC_{0-∞} are in the range of 80% to 125%, range cited in the protocol.

### 90% confidence intervals of celecoxib pharmacokinetic parameters

| Parameter** | Classic Interval | | Power |
|---|---|---|---|
| ABC₀₋ₜ | 96.7440 | 106.0750 | 0.9966 |
| ABC_{0-∞} | 96.6131 | 106.2816 | 0.9956 |
| Cmax | 98.3215 | 120.4409 | 0.9403 |

| | | | |
|---|---|---|---|
| "Logarithmic transformation base e. | | | |

### 90% confidence intervals of acetaminophen pharmacokinetic parameters.

| Parameter** | Classic Interval | | Power |
|---|---|---|---|
| ABC₀₋ₜ | 97.1303 | 104.3860 | 0.9988 |
| ABC_{0-∞} | 97.2555 | 104.8761 | 0.9980 |
| Cmax | 101.6881 | 117.7694 | 0.8877 |

| | | | |
|---|---|---|---|
| "Logarithmic transformation base e. | | | |

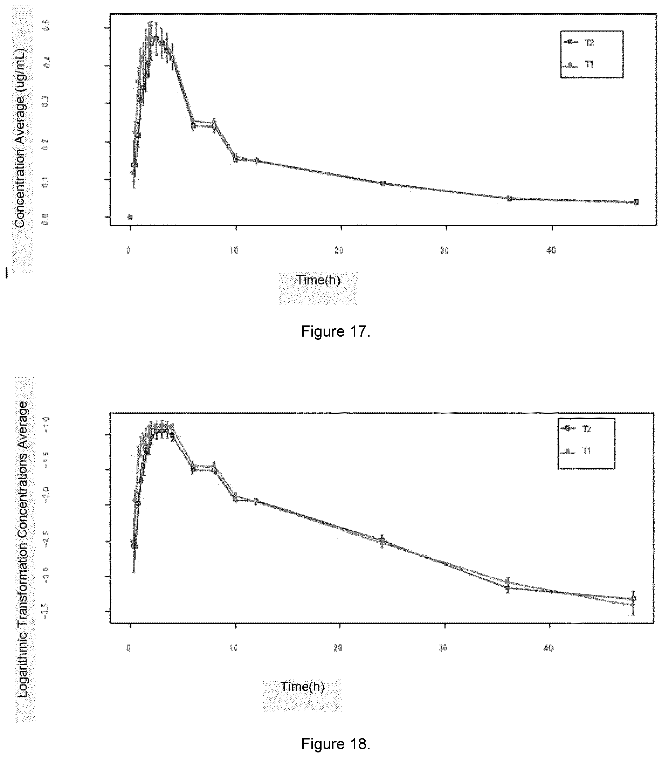

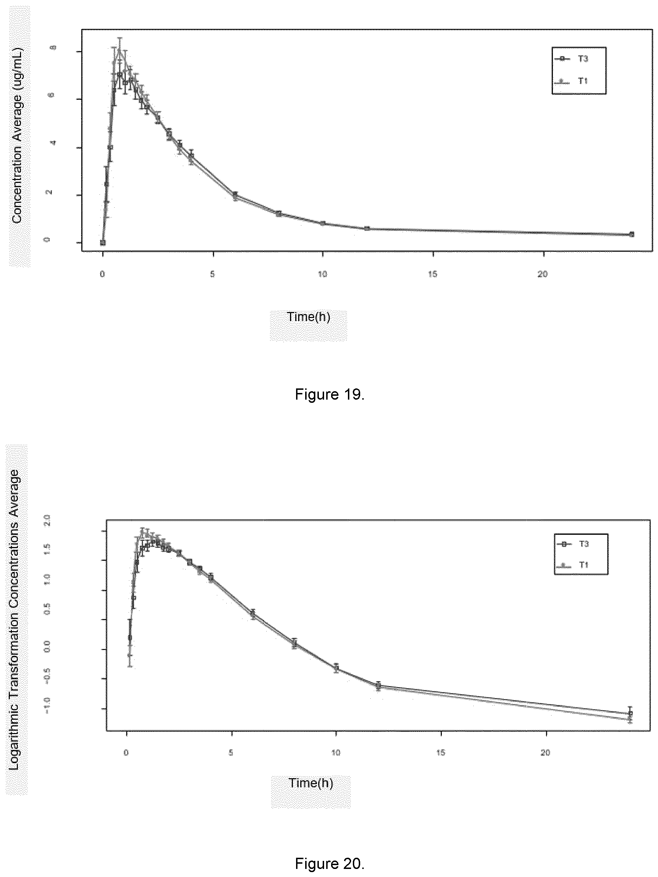

See Figures 17 to 20 for graphs of average plasma concentrations.

### Statistical conclusion

According to the comparative bioavailability tests used and suggested by the NOM-177-SSA1-2013 standard, in celecoxib/acetaminophen tablets of 200 mg/500 mg, respectively, the 90% confidence intervals (IC90) estimated from the logarithmic transformation of the pharmacokinetic parameters Cₘₐₓ, ABC₀₋ₜ, are in the range 80% to 125%, range cited in the protocol; as additional information, the pharmacokinetic parameter for ABC_{0-∞} is in the same range. Likewise, the P-values of t1 and t2 of the Schuirmann double-tailed t test were less than 0.05, allowing the rejection of the null hypothesis of non-equivalence in bioavailability.

From the above it can be concluded that the comparative bioavailability between celecoxib-acetaminophen tablets of 200 mg/500 mg, respectively, manufactured, distributed and packaged by Laboratorios Silanes SA de CV is statistically equivalent to that of the individual medicaments celecoxib capsules of 200 mg (Celebrex ^{®}), manufactured by Pfizer Pharmaceuticals LLC, and acetaminophen tablets of 500 mg (Tylenol ^{®}) manufactured by Janssen- Cilag, SA de CV, which indicates that the speed and the amount absorbed are not affected in the combination celecoxib-acetaminophen tablets of 200 mg/500 mg, respectively. The power of the test is greater than 80% for the parameters evaluated, both for celecoxib and paracetamol.

### Application and advantages of the invention

This invention provides a manufacturing process in which, surprisingly, the selection of unit operations, the order and the execution time of each stage, are essential to control the different physicochemical properties of the drugs, ensuring their long-term conservation stability and their dissolution, so that it complies with the established quality characteristics, guaranteeing the identity, purity and potency of the product, so as to ensure its suitability for use.

The variables that improved for celecoxib include its rheological and adhesive characteristics, and for acetaminophen the rheological and compaction characteristics were improved, ensuring the proper functioning of the product.

The pharmaceutical form selected for dosing the drugs was tablets due to their dosage accuracy. They can also be designed in the form of a caplet because they are easier to swallow and are the smallest possible size for the quantity and weight of the drugs.

An additional advantage of the invention is that, by improving the stability of the product, the shelf life of the medicament is increased, and adverse reactions are reduced, as demonstrated in the phase I clinical study that presents the incidences of adverse effects.

## Claims

1. A pharmaceutical composition comprising celecoxib and acetaminophen as active ingredients, wherein the pharmaceutical composition is an immediate release coated tablet.

2. The pharmaceutical composition according to claim 1, further comprising at least one of a surfactant/humectant agent, a binder, a diluent, a diluent, a disintegrant, a slip, an alkalinizing, a lubricant, a coating, and a vehicle.

3. The composition according to claim 2, further comprising:
- sodium lauryl sulfate as a surfactant/humectant agent;
- polyvidone as a binder;
- microcrystalline cellulose as diluent;
- croscarmellose sodium as disintegrant;
- colloidal silicon dioxide as slip;
- sodium bicarbonate as alkalinizing;
- magnesium stearate as a lubricant,
- a hydroxypropyl methylcellulose coating system;
- purified water as a vehicle.

4. The pharmaceutical composition according to any one of claims 1 to 3, wherein the acetaminophen is present in an amount of 200 mg or 500 mg and celecoxib is present in an amount of 200 mg.

5. The pharmaceutical composition according to any one of claims 1 to 4, wherein the pharmaceutical composition it is adapted to be orally administrable.

6. The pharmaceutical composition according to any one of claims 1 to 5, comprising:
- 200.00 mg of celecoxib;
- 500.00 mg of acetaminophen;
- 17.00 mg of sodium lauryl sulfate as surfactant/humectant;
- 26.50 mg polyvidone as binder;
- 296.50 mg microcrystalline cellulose as diluent;
- 50.00 mg croscarmellose sodium as disintegrant;
- 5.00 mg colloidal silicon dioxide as slip;
- 100.00 mg of sodium bicarbonate as alkalinizing;
- 5.00 mg of magnesium stearate as a lubricant;
- 24.50 mg of a hydroxypropyl methylcellulose coating system;
- 0.635 mL of purified water as vehicle.

7. The pharmaceutical composition according to any one of claims 1 to 5, comprising:
- 200.00 mg of celecoxib;
- 200.00 mg of acetaminophen;
- 11.45 mg sodium lauryl sulfate as a surfactant/humectant;
- 10.60 mg of polyvidone as binder;
- 105.00 mg microcrystalline cellulose as diluent;
- 28.50 mg croscarmellose sodium as disintegrant;
- 2.00 mg colloidal silicon dioxide as slip;
- 56.85 mg of sodium bicarbonate as alkalinizing;
- 2.00 mg of magnesium stearate as a lubricant;
- 12.25 mg of a hydroxypropyl methylcellulose coating system;
- 0.318 mL of purified water as vehicle.

8. A process for manufacturing a stable pharmaceutical composition as that described in any one of claims 1 to 7, comprising the following steps:
a) mixing the active ingredients celecoxib and acetaminophen with at least a humectant, a binder, a disintegrant, a glider and a diluent for approximately 7 minutes;
b) moistening with purified water and granulating the mixture of the previous step;
c) drying the granulate obtained in the previous step until reaching a humidity between approximately 1.8% and 2.0%;
d) adding a binder and a disintegrant, mixing for approximately 5 minutes;
e) moistening with purified water and performing a second granulation;
f) drying the granule obtained in the previous step until reaching a humidity between approximately 1.3% and 1.5%;
g) reducing the size of the granules;
h) adding a disintegrant, an alkalizing and a diluent, mixing for approximately 5 minutes;
i) adding a lubricant and mixing for approximately 3 minutes;
j) compressing the mixture obtained in the previous step cores;
k) coating the cores obtained in the previous with 2.0% coating.

9. The process of claim 8, wherein:
- in step a), 50% of the total amount of the binder, 37.5% of the total amount of the disintegrant and 30.0% of the total amount of the diluent is mixed;
- in step d), the remaining 50% of the binder and 37.5% of the total amount of the disintegrant is added;
- in step h) the remaining 25% of the disintegrant and the remaining 70% of the diluent is added and mixed.

10. The process according to claim 8 or 9, wherein the humectant is sodium lauryl sulfate, the binder is polyvidone, the diluent is microcrystalline cellulose, the disintegrant is croscarmellose sodium, the slip is colloidal silicon dioxide, the alkalinizing is sodium bicarbonate, the lubricant is magnesium stearate, and the coating system is hydroxypropylmethylcellulose.

11. The process in accordance any one of claims 8 to 10, wherein the granulation is wet granulation.

12. The process according to any one of claims 8 to 10, wherein for a pharmaceutical composition comprises a dosage of 200 mg of celecoxib and 500 mg of acetaminophen, the tablet cores have a weight of 1200 mg ± 60 mg.

13. The process according to any one of claims 8 to 10, wherein for a pharmaceutical composition comprises a dosage of 200 mg of celecoxib and 200 mg of acetaminophen, the tablet cores have a weight of 600 mg ± 30 mg.

14. A pharmaceutical composition as described in any one of claims 1 to 7, for use in the treatment of pain.

15. The composition for use according to claim 13, wherein the pain is selected from the group consisting of acute pain and postoperative pain.
